(19) Europäisches Patentamt
European Patent Office
Office européen des brevets

(11) EP 3 328 393 B1

(12) **EUROPEAN PATENT SPECIFICATION**

(45) Date of publication and mention of the grant of the patent:
**20.12.2023 Bulletin 2023/51**

(21) Application number: **16833615.4**

(22) Date of filing: **29.07.2016**

(51) International Patent Classification (IPC):
***A61K 47/68*** *(2017.01)* ***C07K 16/28*** *(2006.01)*
***C07K 16/30*** *(2006.01)* ***A61K 31/585*** *(2006.01)*
***A61P 35/00*** *(2006.01)* ***A61P 29/00*** *(2006.01)*
***A61K 31/704*** *(2006.01)*

(52) Cooperative Patent Classification (CPC):
**A61K 47/6849; A61K 31/585; A61K 47/6803; A61K 47/6807; A61P 29/00; A61P 35/00; C07K 16/2887; C07K 16/3061**

(86) International application number:
**PCT/US2016/044729**

(87) International publication number:
**WO 2017/023760 (09.02.2017 Gazette 2017/06)**

(54) **EXTRACELLULAR DRUG CONJUGATES TARGETING CD20**

AUF CD20 ABZIELENDE EXTRAZELLULÄRE ARZNEIMITTELKONJUGATE

CONJUGUÉS DE MÉDICAMENTS EXTRACELLULAIRES CIBLANT CD20

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.07.2015 US 201562199622 P**
**17.08.2015 US 201562206177 P**
**30.06.2016 US 201662357078 P**

(43) Date of publication of application:
**06.06.2018 Bulletin 2018/23**

(73) Proprietor: **Centrose, Llc**
**Madison, WI 53717-1944 (US)**

(72) Inventor: **PRUDENT, James R.**
**Madison, WI 53717 (US)**

(74) Representative: **Wakerley, Helen Rachael**
**Reddie & Grose LLP**
**The White Chapel Building**
**10 Whitechapel High Street**
**London E1 8QS (GB)**

(56) References cited:
**WO-A1-2014/176284**
**US-A1- 2005 180 972**
**US-A1- 2008 027 010**
**US-A1- 2008 171 040**
**US-A1- 2013 230 517**
**US-A1- 2014 193 436**
**US-A1- 2015 141 414**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### FIELD OF THE DISCLOSURE

The present disclosure provides extracellular drug conjugates useful in the treatment of disease and as tools for the evaluation of biological systems. The present disclosure relates to the fields of biology, chemistry, medicinal chemistry, medicine, molecular biology, and pharmacology.

### BACKGROUND

All fundamental biological processes, including development, immunity, and tumorigenesis, are related to the selective and differential expression of genes in different tissues and cell types. The formation of many malignant tumors has been shown to be associated with the production and/or expression or increased production and/or expression of certain specific cell surface signaling molecules. One of the goals of modern molecular medicine is to find ways to target drugs selectively to reduce or eliminate the drug's off target toxic effects. Delivering drugs to a specific target that is unique to or expressed at higher levels in diseased cells types using targeting moieties such as antibodies, peptides or aptamers has been investigated. Attaching these types of targeting moieties directly to a drug through linkers or to nanoparticles has also been investigated.

A relatively new approach (see PCT Pub. No. 2011/031870 and US Patent Nos. 8,470,980 and 9,364,554) involves the use of agents that have the following structure:

$$\text{[TARGETING MOIETY]}-\left(\text{[LINKER]}-\text{[DRUG]}\right)_n$$

Formula (I)

comprising three portions: a targeting moiety portion (e.g., [TARGETING MOIETY] in Formula (I)), a non-cleavable linker portion (e.g., [LINKER] in Formula (I)), and a therapeutic - or diagnostic - agent portion (e.g., [DRUG] in Formula (I)). As shown in Formula (I), more than one linker-drug may be associated with a single targeting moiety. Accordingly, in some cases, n may be, on average, about 1 to about 10, such as about 4 to about 8. Among the novel aspects of this approach is the key criteria that the targeting moiety and drug both act on - also referred to herein as "bind to" - extracellular targets; hence, such a conjugate is termed an extracellular-targeted drug conjugate (EDC).

Other novel aspects included the nature and length of the linker: the linker is intended to remain intact for the duration of desired therapeutic effect: it is not intended to be cleaved (to release free drug or free targeting moiety) prior to the EDC initiating the desired therapeutic effect by binding both its targets contemporaneously and the length is such to ensure the two targets are on the same cell and in the same complex of proteins on the surface of that cell. While the approach as initially described encompassed a wide variety of suitable agents for the targeting moiety and drug and complementary cell surface targets, one EDC extensively tested and described was composed of an antibody targeting the dysadherin subunit of the Na,K-ATPase (the targeting moiety) with a cardiac glycoside drug (see PCT Pub. No. 2010/017480 and US Patent No. 8,703,723) that had activity as an anti-cancer agent. Subsequent work (see PCT Pub. No. 2012/122514) has shown that such agents (referred to as "EDC1" agents) have promising activity in animal models of cancer, including, for example, in combination with gemcitabine in pancreatic cancer models. Other disclosures include US 2014/193436, a conjugate having a targeting portion, a PEG spacer and scillarenin.

Interesting new discoveries arose out of this work on EDC1 agents (see PCT Pub. No. 2012/178173). Excitingly, one discovery showed the Na,K-ATPase, a complex multi-subunit protein present in various forms in incredibly diverse cell types both normal and diseased, was associated with other cell surface proteins. The resulting complexes exert concerted control over vital cell processes and offer exciting new targets for EDCs in which the drug portion binds the Na,K-ATPase, as in the EDC1 class of drugs, but the targeting moiety portion binds another cell surface protein (instead of a subunit of the Na,K-ATPase as in the EDC1 class of drugs) associated with the former. Such complexes were postulated to create or contribute to diseased states, as suggested by the EDCs specifically exemplified.

Among the targeting moieties specifically identified as associated with the Na,K-ATPase, EDCs targeting CD147 were shown to have promising activity as anti-cancer agents. Subsequent work (see PCT Pub. No. 2015/123687) has identified CD38 as another such Na,K-ATPase associated cell surface signaling protein, in that EDCs targeting the Na,K-ATPase with a drug and CD38 with an antibody are potent and specific anti-cancer agents in vitro. CD20 was identified as a cell surface protein in these publications but there was no data to demonstrate it actually complexed with the Na,K-ATPase or that EDCs targeting it could kill cancer cells specifically.

CD20 is a molecule present on the B lymph cell surface and expression thereof is seen in normal B cells in peripheral blood, spleen, tonsil and bone marrow and so forth as well as B cells in most of malignant tumors. CD20 is highly expressed by over 95% of B cell lymphocytes throughout their development, from the pre-B cell stage until their final differentiation into plasma cells but is absent on the hematopoietic stem cell. CD20 comprises 297 amino acid residues, passes through the cell membrane four times, has both the C-terminus and N-terminus inside the cell, and has one extracellularly exposed loop consisting of 43 amino acid residues between the third and fourth transmembrane domains. Because the CD20 protein is not secreted out of the cell or cleaved, it is considered a non-internalizing plasma membrane but, as noted above, has not previously been shown to be associated with the Na,K-ATPase in any disease state.

CD20 itself, however, has been validated as a therapeutic target for treatment of B cell malignancies. Multiple antibody drug candidates have been developed that target CD20. Monoclonal antibodies that recognize the CD20 antigen include, B1, 1F5, 2H7, and the clinically approved rituximab, first named 2B8 (MabThera®; Rituxan®, Roche/Genentech/Biogen IDEC), which is a chimeric antibody, ofatumumab (Arzerra®; Genmab/GlaxoSmithKline), which is a human antibody approved for refractory CLL, and tositumomab (Bexxar®, GlaxoSmithKline), and ibritumomab tiuxetan (Zevalin®, Spectrum), which are murine antibodies used clinically as radioimmunoconjugates.

Of all the CD20 specific antibodies, rituximab (RITUXIN®, MABTHERA, ZYTUX) is the most widely used clinically. Rituximab has been established as a standard therapeutic agent for low malignancy non-Hodgkin's lymphoma (NHL) and found to have therapeutic effects on many B cell-mediated immunological diseases such as chronic lymphatic leukemia, autoimmune diseases in which a pathogenic autoantibody appears to be involved such as autoimmune hemolytic anemia and idiopathic thrombocytopenia purpura, and inflammatory diseases such as chronic rheumatoid arthritis and multiple sclerosis.

Anti-CD20 antibodies have a property of targeting and eliminating all B cells including normal cells. However, with all the attempts over the past few decades, there are no examples showing anti-CD20 antibodies displaying efficient direct cytotoxicity. In addition, there are no examples of anti-CD20 antibody drug conjugates containing uncleavable linkers that efficiently kill CD20 positive cells [Cancer Res 2009; 69: (6). March 15, 2009]. Development of anti-CD20 antibody targeted drugs showing efficient direct cell killing is desired.

Another disadvantage of anti-CD20 antibodies is their overall performance in human patients with NHL. The effective rate of rituximab alone against recurrence of low malignancy NHL was less than 50% in a clinical study in the United States. The response rate in patients with moderate malignancy NHL is even lower, about 30%. Interestingly, the reason for this poor overall performance is most likely not due to genetic mutations in the rituximab epitope - such could reduce the binding and efficacy of the antibody, because clinical data with DLBCL (diffuse large B cell lymphoma) suggest that epitope mutations are very rare (0.4% of 264 patients at diagnosis and one of 15 patients at relapse) and not an important cause of treatment failure when rituximab is used in combination with conventional chemotherapy (see Haematologica 2009; 94:423-7; PMID:19211644).

There remains a need for new extracellular-targeted drug conjugates (EDC) for the treatment of disease, particularly diseases treatable currently with CD20-targeted therapies for cancer and other disease. The technology of the present disclosure meets that need.

## SUMMARY

The present disclosure generally relates to an EDC of Formula 1 in which the Targeting Moiety is an anti-CD20 antibody, including any anti-CD20 antibody identified herein, the Linker is a non-cleavable linker, and the Drug is a compound of Formula II, as set forth in the Detailed Description below. The invention is as set out in the accompanying claims.

The present disclosure relates generally to EDCs for use in the treatment of diseases and conditions treatable with rituximab or any other disease for which an anti-CD20 antibody or fragment thereof or other binding moiety specific for CD20 is efficacious with an EDC of the invention. Such diseases include, without limitation, leukemias and any B cell-mediated immunological disease, including chronic lymphatic leukemia; lymphomas, such as non-Hodgkin's lymphoma (NHL) generally and diffuse large B cell lymphoma (DLBCL) and low malignancy NHL; autoimmune diseases in which a pathogenic autoantibody appears to be involved such as autoimmune hemolytic anemia and idiopathic thrombocytopenia purpura; and inflammatory diseases such as chronic rheumatoid arthritis and multiple sclerosis. In each of these diseases, EDCs of the invention can be administered for therapeutic effect. Administration may be by intravenous infusion at frequencies typical for other antibody-based drugs, including, for example and without limitation, once a week, twice a month, once every three weeks, and the like.

The present disclosure relates specifically to a class of EDCs, termed the EDC9 class, in which the Targeting Moiety is rituximab or a Rituxan biosimilar, the Linker is a non-cleavable linker such as a PEG linker 24 (PEG24) to 36 (PEG36) monomers in length, and the Drug is a cardiotonic steroid or cardiac glycoside, including for example CEN09-106 (see Figures 1A-1G).

In another aspect, the present disclosure relates generally and specifically to compositions including pharmaceutical formulations and unit dose forms and drug delivery systems comprising EDC consistent with Formula (I) as disclosed herein that are useful in the treatment of disease. In various embodiments, the pharmaceutical formulations are suitable for IV administration.

These and other aspects and embodiments of the present disclosure are described in detail below, after a brief description of the accompanying figures.

BRIEF DESCRIPTION OF THE FIGURES

Figures 1A-1G show structures of representative Drugs and Drug-Linker moieties, and EDC9 conjugates useful in various embodiments of the present disclosure, including forms suitable for use in drug manufacturing and disease treatment. The structures are arranged as follows: Figure 1A shows illustrative cardiotonic steroids in their aglycone form.

Figure 1B shows aminoglycosides of illustrative cardiotonic steroids in free amine form (not linked to linker).

Figure 1C shows illustrative drug moieties covalently attached to heteroatomic portions of a Linker. In Figure 1C, the heteroatomic portions of the Linker are depicted in free amine form. However, under physiological pH, at least one nitrogen atom of each heteroatomic Linker portion conveys a net positive charge within 6 to 10 atomic bonds of the C3 "X" atom of the Drug moiety [of formula (II) or formula (III)].

Figure 1D shows illustrative Drug-Linker compounds modified to be coupled to reduced thiols of an antibody or antibody fragment via maleimide coupling.

Figure 1E shows an illustrative Drug-Linker compound modified to be coupled to an antibody or antibody fragment via aldehyde coupling.

Figure 1F shows an illustrative example of an EDC of Formula (I) with the [TARGETING MOIETY]-[L!NKER]-[DRUG] being an [antibody]-[PEG36]-[CEN09-106], respectively - the conjugation achieved through reaction of CEN10-131 to reduced cysteine thiols.

Figure 1G shows an illustrative example of an EDC of Formula (I) with the [TARGETING MOIETY]-[L!NKER]-[DRUG] being an [antibody]-[PEG36]-[CEN09-106], respectively - the conjugation achieved through reaction of CEN-332 to aldehyde groups produced by oxidization of N-linked glycans on the Fc region in the CH2 domain.

Figure 2 is a graph showing cytotoxic activity of two different EDCs of the invention. The EDCs differ only with respect to the anti-CD20 antibody: the EDC9 conjugate that produced the data in the graph identified as Rituximab-CEN10-131 utilized rituximab as the targeting moiety, and the EDC9 conjugate corresponding to the anti-CD20(2H7)-CEN10-131 data utilized a murine monoclonal antibody (Clone 2H7) as the targeting moiety. In both EDCs, the Drug is CEN09-106 and the Linker includes PEG36. The results shown are from in vitro assays conducted as described in Example 1 against the CD20 positive cell line Ramos, derived from a human Burkitt’s lymphoma cell that is generally resistant to Rituximab in vitro in the absence of human serum complement which elicits complement-dependent cytotoxicity (CDC). In the graph, the x-axis is the log of the IC50 (M), and the y-axis is "relative luminescence units" (RLU), essentially a measure of cellular ATP levels that is the output of the assay used to demonstrated inhibition of cell division or absence of living cells. As shown in the figure and discussed in more detail below in Example 2, the results show the EDC9 conjugates are cytotoxic with IC50s in the picomolar range. As those skilled in the art will appreciate from the graph, the EDCs in this study reduced ATP levels to zero at concentrations at which rituximab itself exerts maximal cytostatic effect.

Figure 3 is a graph (generated as per the graph in Figure 2) showing cytotoxic activity of another EDC9 conjugate consistent with the present disclosure. The data originates from Rituximab-CEN-375), in which the Targeting Moiety is rituximab, the Drug is CEN371, and the Linker includes PEG24. The results demonstrate that this EDC9 species is a potent cytotoxic agent, as is more fully discussed in Example 3, below.

Figure 4 is a graph showing cytotoxicity of Rituximab-CEN10-131 and Rituximab-CEN375 EDC9 species against the CD20-positive cell line SUDHL4, derived from a Non-Hodgkin’s lymphoma cell. The results again show that the EDC9 conjugates are more potent than rituximab, and cytotoxic rather than cytostatic under these test conditions, as is more fully discussed in Example 4, below.

Figure 5 is a three dimensional bar chart showing that the EDC9 conjugate Rituximab-CEN10-131 is essentially non-toxic to normal human peripheral blood mononuclear cells (PBMCs) at concentrations in which 100% of the Ramos and SUDHL4 cells are killed. The z-axis shows the percent living cells at the readout; the y-axis shows the EDC concentration used for the corresponding readout; and the x-axis shows the cell line used to generate the readout. The results are more fully discussed in Example 5, below.

Figures 6A and 6B show the results of xenograft studies using an EDC9 conjugate of the present disclosure. Figure 6A is a graph of the results of a study comparing Rituximab-CEN10-131 to rituximab at the same 5 mg/kg doses

using the dosing schedule of q6dx2 via i.p. injection in CB17SCID mice (n = 6 per group) bearing SU-DHL-4 xenograft tumors of an average size of 250 $mm^3$ starting at day 21 post tumor implant: vehicle (dashed line), rituximab (diamond), or Rituximab-CEN10-131 (circle). The y-axis is average tumor volume ($mm^3$), and the x-axis is days post tumor implant. The data shows that Rituximab-CEN10-131 was superior to rituximab in repressing tumor growth during the study, with rituximab demonstrating activity as expected over vehicle alone. Figure 6B shows the results of a similar study in CB17SCID mice (n = 6 per group) bearing s.c. SU-DHL-4 xenograft tumors of an average size of 250 $mm^3$ in which Rituximab-CEN10-131 was dosed at 0.5 mg/kg (triangle), 1.5 mg/kg (square),or 5 mg/kg (circle) using the dosing schedule of q6dx2 via i.p. injection starting at day 21 post tumor implant. The results demonstrate a clear dose dependence in inhibition of tumor growth. Data in Figure 6A-6B are plotted as group mean tumor volumes post tumor implant, with standard error of the mean (SEM) indicated by vertical bars. The results are more fully discussed in Example 6, below.

Figures 7A-7C show the results of a study demonstrating EDCs of the present disclosure are efficacious in models using larger tumors than those used in the studies shown in Figure 6. In this study, Rituximab-CEN10-131 or a control EDC (poly clonal human IgG conjugated to CEN10-131) was administered to mice bearing SUDHL4 tumors of an average size of 500 $mm^3$. Figure 7A shows a tumor growth curve (tumor volume versus time in days) of groups of mice (n=11/group) treated with a single dose at 25 mg/kg of Rituximab-CEN10-131 or control EDC, plus a vehicle-only control group. Figure 7B shows average post-implant weight of the mice for the same study. Figure 7C shows survival analysis (% of animals in each group with a tumor volume <2000$mm^3$ over time in days) for the same study. The results demonstrate that Rituximab-CEN10-131 is highly efficacious and specific, as CD20 targeting was required for the EDC to be cytotoxic (control EDC showed no activity) are more fully discussed in Example 7, below.

Figure 8 shows the results of a study in which the EDC9 conjugate Rituximab-CEN10-131, a control EDC that does not bind CD20, and various fragments of the EDC9 conjugate (alone or in combination with rituximab) as well as a rituximab control were tested in an in vitro assay to determine their EC50 values on Ramos cells (in the absence of complement). The various fragments tested were: the cardiac glycoside drug moiety (CEN09-106); CEN10-131, which corresponds to the maleimide-activated drug-linker portion of Rituximab-CEN10-131; and an unconjugated mixture of rituximab and CEN10-131. On a molar basis, the EDC9 conjugate Rituximab-CEN10-131 was 25X more potent than the drug (CEN09-106) and 2600X more potent than the drug-linker (CEN10-131). The results show that Rituximab-CEN10-131 is a potent cytotoxic agent (122 pM EC50), with the drug showing significant but

much less potent activity, and the drug linker, rituximab, and mixture of the two showing essentially no activity. The equimolar unconjugated mixture of rituximab and CEN10-131 drug-linker (no covalent attachment between the two) result demonstrates that the drug linker must be covalently attached to the targeting moiety-as in Rituximab-CEN10-131-to achieve the potency and selectivity seen with the latter. The results are more fully discussed in Example 8, below.

## DETAILED DESCRIPTION

The present disclosure relates generally to Extracellular-targeted Drug Conjugates or EDC in which the agent moiety (which is a therapeutic agent such as a drug, according to the claims, but is generally referred to as "drug" herein) and targeting moiety (which is an anti-CD20 antibody or binding fragment thereof) bind to or act on complexes containing the Na,K-ATPase (encoded by the ATP1 family of genes, including, for example the ATP1A1, ATP1A2, ATP1A3, and ATP1A4 genes). The EDC are useful in a variety of applications, particularly the treatment of human disease, such as cancer. More specifically, the EDC are useful in the treatment of leukemias and any B cell-mediated immunological disease, including chronic lymphatic leukemia; lymphomas, such as non-Hodgkin's lymphoma (NHL) generally and diffuse large B cell lymphoma (DLBCL) and low malignancy NHL; autoimmune diseases in which a pathogenic autoantibody appears to be involved such as autoimmune hemolytic anemia and idiopathic thrombocytopenia purpura; and inflammatory diseases such as chronic rheumatoid arthritis and multiple sclerosis, and have application in lung disease, including asthma and other diseases involving inflammation and other medical conditions. In the EDC of the present disclosure, the agent moiety is a drug that binds or otherwise blocks or interferes with the ion channel of the Na,K-ATPase, and the targeting moiety is an antibody or antibody fragment that specifically binds human CD20.

The structures of Formula (I) and Figures 1F-1G are provided for clarity to show an illustrative [Targeting Moiety]-[Linker]-Drug] in an EDC that will typically have more than one such linkage (e.g., one target loading is 4 Drugs per Antibody). The compounds and structures of Figures 1A-1E, however, are intended to depict compounds utilized in synthetic procedures of the invention, including both known starting materials and structures and novel intermediates of the EDCs of the invention. One of skill in the art will recognize that the illustrated intermediates may also be useful in making other EDCs, such as those that bind to extracellular targets other than CD20, which are not according to the invention.

For convenience, and because the glycoside moieties are optional in the "drugs" of the disclosure, some structures are illustrated with any glycoside moiety in the linker, and the drug is shown without any glycoside. However,

in Figures 1A-1G, the glycoside moiety may alternatively be shown or referred to in the context of the "drug" portion of the EDC being described, for example because starting materials and/or intermediate compounds include cardiac glycoside drugs. These alternate representations should not be interpreted as affecting the scope of the inventive class of EDC9 conjugates described herein.

I. Definitions

The term "amino acid" refers to naturally occurring and non-natural amino acids, as well as amino acid analogs and amino acid mimetics.

The term "antibody" refers to a protein or mixture of proteins that comprise one or more peptidic chains encoded by immunoglobulin genes or fragments thereof that specifically bind and recognize an epitope of an antigen. The recognized immunoglobulin genes include the kappa, lambda, alpha, gamma, delta, epsilon, and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively. Typically, the antigen-binding region of an antibody will be most critical in specificity and affinity of binding. The antibodies comprise IgG (including IgG1, IgG2, IgG3, and IgG4), IgA (including IgA1 and IgA2), IgD, IgE, or IgM, and IgY. As used herein, the term "antibody" is meant to include whole antibodies, including single-chain antibodies, and antigen-binding fragments thereof. Antibodies can also be antigen binding antibody fragments and include, but are not limited to, Fab, Fab' and F(ab')2, Fd, single-chain Fvs (scFv), single-chain antibodies, disulfide-linked Fvs (sdFv), diabodies, triabodies, tetrabodies, minibodies, and fragments comprising either a VL or VH domain, and Nanobodies (see PCT publication number WO 94/04678 and Nature Medicine, V9 (1) pp 129-134, 2003). An antibody can be from any animal origin including birds and mammals. Typically, antibodies in commercial or research use are human, murine, rabbit, goat, guinea pig, camelidae (e.g., camel, llamas), horse, or chicken antibodies. "Antibodies", as used herein, includes monoclonal, chimeric, and humanized antibodies, as well as intact antibodies and isolated antibodies. Antibodies can be monospecific, bispecific, trispecific or greater multispecificity. The term "extracellular-targeted drug conjugate" or "EDC" refers to a drug conjugate in which an antibody or other targeting moiety that targets an extracellular target is linked via a stable or non-cleavable linker to a drug that binds to an extracellular target.

The term "antigen" refers to the substance or target that an antibody or targeting moiety binds. An antigen is characterized by its ability to be "bound" by the antibody or targeting moiety. Antigen can also mean the substance used to elicit the production of targeting moieties, such as the production of antigen specific antibodies through immunizing with the antigen.

The term "antigen binding site" or "epitope" refers to the portion of the antigen to which an antibody binds.

The term "binding affinity" refers to the strength of interaction between an antibody (or other targeting moiety or drug or other agent) and its antigen (or target) as a function of its association and dissociation constants. Higher affinities typically mean that the targeting moiety has a fast on rate (association) and a slow off rate (dissociation). Binding affinities can change under various physiological conditions and changes that occur to the antigen or antibody/targeting moiety under those conditions. Binding affinities of the targeting moiety can also change when therapeutic agents and/or linkers are attached. Binding affinities can also change when slight changes occur to the antigen, such as changes in the amino acid or glycosylation of the antigen.

The term "cancer" refers to any of a number of diseases characterized by uncontrolled, abnormal proliferation of cells, the ability of affected cells to spread locally or through the bloodstream and lymphatic system to other parts of the body (i.e., metastasize), as well as any of a number of characteristic structural and/or molecular features. A "cancerous cell" or "cancer cell" is understood as a cell having specific structural properties, which can lack differentiation and be capable of invasion and metastasis. Examples of cancers are, breast, lung, brain, bone, liver, kidney, colon, and prostate cancer (see DeVita, V. et al. (eds.), 2005, Cancer Principles and Practice of Oncology, 6th. Ed., Lippincott Williams & Wilkins, Philadelphia, PA).

The term "chimeric antibodies" refers to antibodies in which the Fc constant region of a monoclonal antibody from one species (typically a mouse) is replaced, using recombinant DNA techniques, with an Fc region from an antibody of another species (typically a human). For example, a cDNA encoding a murine monoclonal antibody is digested with a restriction enzyme selected specifically to remove the sequence encoding the Fc constant region, and the equivalent portion of a cDNA encoding a human Fc constant region is substituted. A CDR-grafted antibody is an antibody in which at least one CDR of a so-called "acceptor" antibody is replaced by a CDR "graft" from a so-called "donor" antibody possessing desirable antigen specificity. Generally the donor and acceptor antibodies are monoclonal antibodies from different species; typically the acceptor antibody is a human antibody (to minimize its antigenicity in a human), in which case the resulting CDR-grafted antibody is termed a "humanized" antibody. The graft may be of a single CDR (or even a portion of a single CDR) within a single VH or VL of the acceptor antibody, or can be of multiple CDRs (or portions thereof) within one or both of the VH and VL. Methods for generating CDR-grafted and humanized antibodies are taught by Queen et al. U.S. Pat. No. 5,585,089, U.S. Pat. No. 5,693,761 and U.S. Pat. No. 5,693,762; and Winter U.S. Pat.

No. 5,225,539.

The term "circulatory structure" refers to body fluids, interstitial fluid, lymph and blood of a mammal, including tissues of the circulatory system.

The term "epitope" refers to groupings of molecules such as amino acid residues or sugar side chains at the surface of antigens that usually have specific three dimensional structural characteristics, as well as specific charge characteristics, and that are capable of specific binding by a monoclonal antibody.

The term "extracellular" refers to the outer surface of a cell membrane.

The term "intact antibody" comprises at least two heavy (H) chains and two light (L) chains interconnected by disulfide bonds. Each heavy chain is comprised of a heavy chain variable region (abbreviated herein as HCVR or VH) and a heavy chain constant region. The heavy chain constant region is comprised of three domains, CH1, CH2 and CH3. Each light chain is comprised of a light chain variable region (abbreviated herein as LCVRX or VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxyl-terminus in the following order: FR1, CDR1, FR2, CDR2, FR3, CDR3, FR4. The variable regions of the heavy and light chains contain a binding domain that interacts with an antigen. The constant regions of the antibodies can mediate the binding of the immunoglobulin to host tissues or factors, including various cells of the immune system (e.g., effector cells) and the first component (Clq) of the classical complement system. Examples of binding fragments include (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., Nature 341: 544-546, 1989), which consists of a VH domain; and (vi) an isolated complementarily determining region (CDR).

The term "heterobifunctional linker" refers to a linker with different reactive groups at either end, enabling sequential conjugation between two different functional groups in proteins and other molecules.

The term "extracellular target" refers to a target, such as a protein, antigen, and/or epitope located on the outer surface of the cell membrane.

The term "linker" refers to a chemical moiety or bond that covalently attaches two or more molecules, such as a targeting moiety and a drug.

The term "linker spacer group" refers to atoms in the linker that provide space between the two molecules joined by the linker.

The term "monoclonal antibody" refers to a preparation of antibody molecules of single molecular composition. A monoclonal antibody composition displays a single binding specificity and affinity for a particular epitope. The term "human monoclonal antibody" refers to antibodies displaying a single binding specificity which have variable and constant regions (if present) derived from human germline immunoglobulin sequences. Human monoclonal antibodies can be produced by a hybridoma which includes a B cell obtained from a transgenic non-human animal, e.g., a transgenic mouse, having a genome comprising a human heavy chain transgene and a light chain transgene fused to an immortalized cell, although the term "monoclonal antibody" is not limited to antibodies produced through hybridoma technology. The term "monoclonal antibody" refers to an antibody that is derived from a single clone, including any eukaryotic, prokaryotic, or phage clone, and not the method by which it is produced. Monoclonal antibodies can be prepared using a wide variety of techniques known in the art including the use of hybridoma, recombinant, and phage display technology.

The term "modified antibodies" refers to antibodies, such as monoclonal antibodies, chimeric antibodies, and humanized antibodies, which have been modified by, e.g., deleting, adding, or substituting portions of the antibody. For example, an antibody can be modified by deleting the constant region and replacing it with a constant region meant to increase half-life, e.g., serum half-life, stability or affinity of the antibody. Multiple molecules of a therapeutic agent or multiple different agents can be coupled to one antibody molecule. For example, different moieties can be coupled to an antibody molecule via the same linker, or multiple linkers that provide multiple sites for attachment (e.g., dendrimers) can be used.

The terms "non-cleaved" and "uncleaved" refer to an EDC composition at any point in time in which the majority (for example, >50%, >60%, >70%, or >80%) of EDC components present are intact, i.e., the linker used to attach the agent to the targeting moieties has not been cleaved.

The term "non-cleavable linker" refers to a stable linker that has the property of being more stable in vivo than either the therapeutic or the targeting moiety under the same physiological conditions. Examples of non-cleavable linkers include linkers that contain polyethylene glycol chains or polyethylene chains that are not acid or base sensitive (such as hydrazone containing linkers), are not sensitive to reducing or oxidizing agents (such as those containing disulfide linkages), and are not sensitive to enzymes that may be found in cells or circulatory system.

The terms "pharmaceutically effective amount" and "effective amount" in the context of an amount of drug delivered refer to an amount of a drug that can induce a desired biological or medical response in a tissue, system,

animal, or human.

The terms "peptide", "polypeptide", peptidomimetic and "protein" are used, somewhat interchangeably, to refer to a polymer of amino acid residues. The terms apply to amino acid polymers in which one or more amino acid residues is an artificial chemical mimetic of a corresponding naturally occurring amino acid, as well as to naturally occurring amino acid polymers. These terms also encompass the term "antibody". "Peptide" is often used to refer to polymers of fewer amino acid residues than "polypeptides" or "proteins". A protein can contain two or more polypeptides, which may be the same or different from one another.

The term "receptor" refers to an extracellular target protein molecule, embedded in either the plasma membrane or the cytoplasm of a cell, to which one or more specific kinds of signaling molecules may bind. Each cell typically has many receptors, of many different kinds.

The term "substantially simultaneously" refers to two or more events that occur at the same time or within a relatively narrow time frame. In various embodiments, substantially simultaneously refers to two or more events that occur within about 60, about 40, about 30, about 20, about 10, about 5, about 2 or about 1 second of each other. For example, EDCs of the invention have properties such that targeting moiety binding and agent (drug) action happen substantially simultaneously.

The term "stable in the circulatory system" refers to the property of a compound, such as an EDC, to resist degradation and means that, for example, less than about 50%, or less than about 20%, or typically less than about 2%, of the compound is degraded or cleaved in the circulating blood at about 37°C for at least about 2 hours.

The term "stable linker" refers to a linker that remains stable and intact until the conjugate has been delivered or transported to the target site - a stable linker remains covalently attached to the two molecules it links - in physiological conditions (at 37°C and pH 7) in vivo or in vitro for a period of time sufficient to allow the EDC to reach the target(s) and bind to the target(s). Thus, a stable linker is generally stable within the circulatory structure (generally means below 5% degradation after at least a 2 hour period and, in some embodiments, at least 4, 8, 16, or 24 hour periods).

The term "synergistically" refers to an effect of two or more agents when used in combination that is greater than the sum of the effects of both agents when used alone. For example, in the EDCs of the invention, the combined therapeutic effects of the interaction of the antibody and the agent (drug) when linked through a linker are greater than the combined individual effects of the targeting moiety and agent when used alone. "Effects" can refer either to binding, therapeutic effect, and/or specificity.

The term "target" refers to the protein, glycoprotein, antigen, carbohydrate or nucleic acid to which a targeting moiety binds and also refers to the protein, glycoprotein, antigen, carbohydrate or nucleic acid to which a therapeutic agent (which may be referred to herein as a "drug") binds. The agent and targeting moiety may bind to different targets in a "target complex", where "target complex" refers to two or more molecules, such as the different subunits of a multi-subunit protein or two different proteins in a multi-protein complex, that are in close physical proximity with one another in vivo.

The term "target cells" refers to the cells that are involved in a pathology and so are preferred targets for therapeutic activity. Target cells can be, for example and without limitation, one or more of the cells of the following groups: primary or secondary tumor cells (the metastases), stromal cells of primary or secondary tumors, neoangiogenic endothelial cells of tumors or tumor metastases, macrophages, monocytes, polymorphonuclear leukocytes and lymphocytes, and polynuclear agents infiltrating the tumors and the tumor metastases.

The interchangeable terms "targeting moiety" and "targeting agent" refer to an antibody that binds specifically to a target.

The term "target tissue" refers to target cells (e.g., tumor cells) and cells in the environment of the target cells.

The terms "therapeutic agent" and "drug" and "agent" are used interchangeably herein to refer to a compound that, when present in a therapeutically effective amount, upon binding to a site of action, produces a therapeutic effect, and whose site of action is located or whose effect will be exerted on the surface or inside target cells.

The term "therapeutic effect" refers to the reduction, elimination, and/or prevention of a disease, symptoms of the disease, or side effects of a disease in a subject.

The terms "treating" and "treatment" are used interchangeably to refer to the administration of a therapeutic agent or composition to a patient who has a disease or disorder (e.g., cancer or metastatic cancer), a symptom of disease or disorder or a predisposition toward a disease or disorder, with the purpose to cure, heal, alleviate, relieve, alter, remedy, ameliorate, improve or affect the disease or disorder, the symptoms of the disease or disorder, or the predisposition toward disease. "Treating" or "treatment" of cancer or metastatic cancer refers to the treatment or amelioration or prevention of a cancer, including any objective or subjective parameter such as abatement; remission; diminishing of symptoms or making the disease condition more tolerable to the patient; slowing in the rate of degeneration or decline; or making the final point of degeneration less debilitating. The treatment or amelioration of symptoms can be based on objective or subjective parameters, including the results of an examination by a physician. Accordingly, the term "treating" includes the administration of a therapeutic agent to prevent or delay, to alleviate, or to arrest or inhibit development of the symptoms or conditions associated with a disease, including for example neoplastic disease.

The term "tumor specific antigen" refers to proteins or other molecules that are unique to a tumor or is at least more abundant on tumor cells, relative to normal cells.

II. EDC9 Conjugate Class of Extracellular Drug Conjugates (EDCs)

In the EDCs of the present disclosure (generally referred to herein as "EDC9 conjugates"), the targeting moiety is linked to an agent (drug) via a stable or non-cleavable linker, one that remains intact for an extended period (hours to days) under the physiologic conditions in which it is intended to exert its therapeutic effect. For the EDC9 conjugates of the present disclosure, the linker has to be intact or non-cleaved, keeping the drug and targeting moiety covalently attached, for the EDC to exert its maximal therapeutic effect. While in many embodiments the linker includes polyethylene glycol (PEG) of various monomer lengths between about 12 (PEG12) and 36 (PEG36), the PEG24 and PEG36 linkers work well, with the PEG36 linker providing the most potent and specific anti-cancer activity in the limited in vitro and animal testing done to date. In various other embodiments, however, the linker comprises one or more heteroatoms such as nitrogen. While the various depictions of EDCs herein that contain glycoside residues are generally depicted as having such residues as part of the drug portion of the EDC, the linker can also have a glycoside, such as an aminoglycoside, component (a particular glycoside monomer might be viewed as part of the linker or part of the drug in the same EDC depending on the nature of the glycoside).

The EDC9 conjugates of the present disclosure act on complexes of the Na,K-ATPase and CD20, with "complexes" intended to indicate that the two are in close physical proximity to one another and interacting in such a manner that disruption of CD20 binding can result in a change in the activity of the complex. In the EDC9 conjugates of the present disclosure, the targeting moiety targets (e.g., binds to) CD20, and is an antibody, i.e., an anti-CD20 antibody or a binding fragment thereof. Excitingly, CD20 antibody-based targeting moieties have therapeutic activity in and of themselves, and the EDC9 conjugates of the present disclosure provide an enhanced therapeutic effect relative to such drugs. In action, the EDC9 conjugates of the present disclosure deliver the CD20 targeting moiety more selectively to target cells and tissues than the targeting moiety administered alone, and make it more toxic when it arrives. In many embodiments, the targeting moiety binds (e.g., specifically binds) to CD20 when the CD20 is associated with the Na,K-ATPase, and modulates the cell signaling pathway in that cell by virtue of the drug moiety, which in many embodiments is a cardiotonic steroid or cardiac glycoside that binds to the Na,K-ATPase (or to a protein binding site that blocks interaction with the Na,K-ATPase) and is attached to the targeting moiety via a stable or non-cleavable linker.

The three portions of the EDC of the present disclosure can thus comprise, consist essentially of or consist of: (1) a targeting moiety that binds to CD20; (2) a stable or non-cleavable linker that connects the targeting moiety to the therapeutic agent (drug) and remains intact (uncleaved) during the time needed for the EDC to bind to its targets (targeting moiety and drug both bind separate but associated targets); and (3) a drug agent that binds to the Na,K-ATPase (or to a site on an associatec protein that controls association of CD20 with the Na,K-ATPase), such as a cardiotonic steroid or cardiac glycoside. In some embodiments, an EDC9 conjugate of the present disclosure may include several (e.g., 1 to about 10, or about 4 to about 8) drug moieties each connected to a single targeting moiety (e.g., an anti-CD20 antibody) by non-cleavable linker moieties, as shown representatively in Formula (I). As such, the EDC9 conjugates of the present disclosure generally include a targeting moiety covalently attached to about 1 to about 10 Drug-Linker intermediates, for example about 4 to about 8 Drug-Linker intermediates, or about 4 to about 6 Drug-Linker intermediates.

III. EDC9 Targeting Moieties

The EDCs of the present disclosure have a targeting moiety that binds to CD20. In some embodiments, the targeting moiety is rituximab (also referred to as IDEC-C2B8 or anti-CD20). In some embodiments, the targeting moiety is a rituximab biosimilar. In some embodiments, the rituximab biosimilar is selected from the group consisting of: GP2013 (Sandos), Reditux (Dr. Reddy's Laboratories), BI695500 (Boehringer Ingleheim), MabTas (Intas Biopharmaceuticals), MK-8808 (Merck), PF-0528050586 (Pfizer), a rituximab biosilimar from Torrent, a rituximab biosilimar from Zenotech, CT-P10 (Celltrion/Hospira), and AcellBia (Biocad).

A wide variety of targeting moieties for CD20 are known. CD20 is a transmembrane cellular protein that has been validated as a therapeutic target for treatment of B cell malignancies. CD20 is highly expressed by over 95% of B cell lymphocytes throughout their development, from the pre-B cell stage until their final differentiation into plasma cells, but is absent on the hematopoietic stem cell. Multiple antibody drugs have been developed that target CD20, and any of these antibodies can be used in EDCs of the invention. Monoclonal antibodies that recognize the CD20 antigen include, B1, 1F5, 2H7, and the clinically approved rituximab first names 2B8 (MabThera®; Rituxan®, Roche/Genentech/Biogen IDEC), which is a chimeric antibody, ofatumumab (Arzerra®; Genmab/GlaxoSmithKline), which is a human antibody approved for refractory CLL and tositumomab (Bexxar®, GlaxoSmithKline) and ibritumomab tiuxetan (Zevalin®, Spectrum), which are murine antibodies used clinically as radioimmunoconjugates. Ongoing research aims to develop novel anti-CD20 antibodies with improved properties and greater clinical efficacy, as is the case with obinutuzumab

(GA101), and any such antibodies can also be used to make EDCs of the invention. In all cases, EDCs of the invention will be more potent, safer, and therefore more efficacious treatments of the diseases and conditions for which the antibody alone is efficacious.

In some embodiments, the targeting moiety is an antibody or antibody fragment that binds to CD20 and comprises an Fv sequence similar to Rituximab or to any of the foregoing Rituximab biosimilar agents.

Of all the CD20 specific antibodies, rituximab (RITUXIN®, MABTHERA, ZYTUX) is the most widely used clinically. Rituximab has been established as a standard therapeutic agent for low malignancy non-Hodgkin's lymphoma (NHL) and found to have therapeutic effects on many B cell-mediated immunological diseases such as chronic lymphatic leukemia, autoimmune diseases in which a pathogenic autoantibody appears to be involved such as autoimmune hemolytic anemia and idiopathic thrombocytopenia purpura, and inflammatory diseases such as chronic rheumatoid arthritis and multiple sclerosis. Thus, the results presented herein (see Examples and Figures) are especially exciting in that they demonstrate the invention provides methods for treating these diseases and medicaments useful therein.

There was no data prior to this disclosure that suggested that, on the plasma membrane, the Na,K-ATPase is in close proximity to CD20. Thus, while the prior PCT applications describing EDCs identified CD20 as a cell surface protein, the present discovery that targeting CD20 with an EDC, such as an EDC9 conjugate as disclosed herein, would lead to improved methods for treating diseases treatable with rituximab and other anti-CD20 targeting moieties was surprising and unexpected. The results discussed herein demonstrate that various EDCs within the scope of this disclosure can specifically target and kill tumor cells in a manner superior to that of rituximab or other CD20 targeting moieties not in EDC form.

Surprisingly, in spite of the small molecular size of CD20, targeting moieties to CD20 (e.g., antibodies) show epitope diversity. Therefore, effects other than those specifically described herein may be provided by EDCs falling within the scope of the present disclosure. Without wishing to be bound by theory, it is believed that this diversity may be due to the formation of CD20 specific protein-protein complexes on the plasma membrane. Examples include, down-regulation of B-cell receptors, increases in MHC class II antigens and adhesion molecule expressions, $Ca^{2+}$ release, inhibition of lymphocyte function-associated antigen 1 non-dependent homotypic adhesion, induction of apoptosis, promotion of cell growth. As with the observed improved effects of EDC9 conjugates specifically described in the Figures and Examples of the present disclosure, EDCs of the present disclosure may also provide superior down-regulation of B-cell receptors, increases in MHC class II antigens and adhesion molecule expressions, $Ca^{2+}$ release, inhibition of lymphocyte function-associated antigen 1 non-dependent homotypic adhesion, induction of apoptosis, and/or promotion of cell growth compared to the corresponding antibodies alone. Anti-CD20 antibodies can also have different characteristics and biological functions, and reference to a monoclonal antibody binding to CD20 alone, cannot specify all biological properties. However, EDCs of the invention can be prepared with any anti-CD20 antibody for the purpose of improving therapeutic benefit.

When administered alone, anti-CD20 antibodies generally target and eliminate all B cells, using indirect methods such as CDC or ADCC processes. Sometimes these processes malfunction leading to anti-CD20 antibody resistance. Based on previous publications, the following potential mechanisms might contribute to the resistance: 1) exhaustion of effector cells, such as natural killer (NK) cells, etc. 2) host immunologic factors, such as Fc receptor polymorphisms; and 3) exhaustion of stored complements or increased surface expression of complement control proteins. Therefore, an EDC9 conjugate including an anti-CD20 antibody as the targeting moiety that directly kills B-cells is preferred.

However, with all the attempts over the past few decades, there are no examples showing anti-CD20 antibodies displaying efficient direct cytotoxicity. In addition, there are no examples of anti-CD20 antibody drug conjugates containing uncleavable linkers that efficiently kill CD20 positive cells [Cancer Res 2009; 69: (6). March 15, 2009]. Therefore, development of anti-CD20 antibody targeted drugs showing efficient direct cell killing is an unmet need. Availability of effective treatments for subjects exhibiting anti-CD20 antibody resistance (e.g., Rituximab resistance) has also been unmet; meeting this need has been especially problematic to date. The EDC9 conjugates disclosed herein meet these needs, as demonstrated by the results presented in the Examples and Figures included herein.

Another disadvantage of anti-CD20 antibodies is their overall performance in human patients with NHL. In a clinical study, the effective rate of rituximab alone against recurrence of low malignancy NHL is approximately 50%, indicating that many patients do not respond or respond poorly to rituximab. The response rate in patients with moderate malignancy NHL is even lower, approximately 30%. The reason for this poor overall performance does not appear to be due to genetic mutations because clinical data patients with DLBCL suggest that CD20 epitope mutations are rare [Haematologica 2009; 94:423-7; PMID:19211644]. Therefore, the development of antibody therapies targeting CD20 with direct killing effects is desired.

IV. Drug Moieties

The drug portion comprises a bufadienolide, a cardiac glycoside or an aglycone of a cardiac glycoside. CEN09-106 and other steroids in this class of drugs are useful drugs in the EDCs of the present disclosure. Generally,

the drug portion binds to Na,K-ATPase affecting pump activity (e.g., reduces pump activity or stops pump activity). Typically, the drug portion acts directly on the Na,K-ATPase, e.g. alpha subunit. The Na,K-ATPase is an ion pump found on all cells and consists of three membrane spanning subunits ($\alpha$, $\beta$, and y) each comprising multiple isoforms. Of the three subunits, only the $\alpha$-subunit actively pumps ions in an ATPdependent manner, resides predominantly inside the cell, and is inhibited by cardiac glycoside binding. The EDC9 drug portion binds a deep extracellular pocket within the $\alpha$-subunit inhibiting its activity. In various embodiments of the present disclosure, the drug may be a steroid, a modified steroid, a steroid derivative (e.g., a functionalized steroid), a cardiotonic steroid, a cardiac glycoside, or a cardenolide. In some embodiments, the drug is a bufadienolide such as bufalin, scillarenin, or digitoxigenin. In other embodiments, the drug is a cardiac glycoside or an aglycone of a cardiac glycoside, such as digitoxin, digoxin, ouabain or proscillaridin.

The drug may be a cardenolide, cardiotonic steroid or cardiac glycoside of Formula (II) or Formula (III) below:

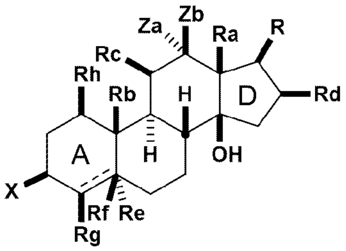

Formula (II)

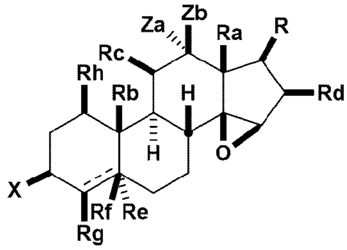

Formula (III)

Za = H,D, F, OH, OAc
Zb = H,D, F, OH, OAc
or Za,Zb = C=O, F2, D2

where the steroidal rings are either saturated, unsaturated or a combination thereof,

R = 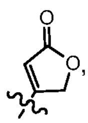, 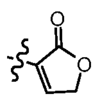, 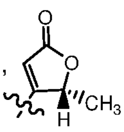, 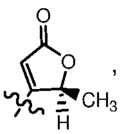, 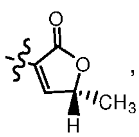, 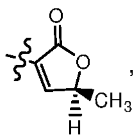, 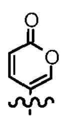, 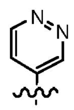, 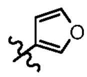 or 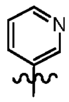

or R = 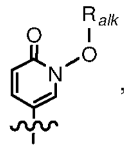, 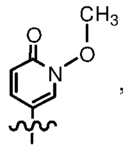, 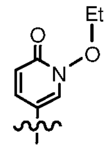, 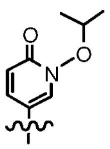, 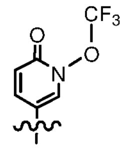, 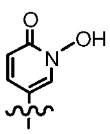 or 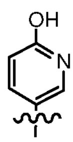.

R can also be H, OH, =O, $C(O)CH_3$, $COCH_2OH$, or a branched alkane. $R_a$ is $CH_3$; $R_b$ is $CH_3$, $CH_2OH$, or CHO; $R_c$ is H, OH or $CH_3COO$; $R_d$ is H, OH or $CH_3COO$; $R_e$ is H or no group; $R_f$ is H, OH or, when $R_e$ is H or a C=C exists between the atoms joined to $R_e$, $R_f$ and $R_g$, $R_f$ is no group; $R_g$ is H or, when a C=C exists between the atoms joined to $R_e$, $R_f$ and $R_g$, $R_g$ is no group; $R_h$ is H or OH; X is O, S or N(OR', SR', NR'), optionally further including a sugar moiety as further discussed below; and R' is an alkyl or aryl group. $R_{alk}$ may be any alkyl group.

In some embodiments, X includes a sugar or modified sugar moiety, such as a pentose, a hexose, an aminopentose, or an aminohexose. In some embodiments, X is chosen from:

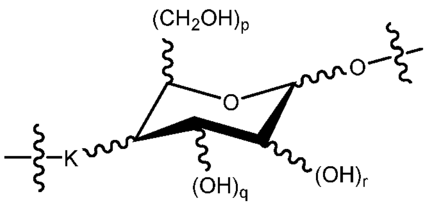,

wherein K is N or O and p, q and r are each independently 0 or 1. In some such embodiments, X is a D- or L- configuration of hexose, pentose, deoxyhexose, deoxypentose, deoxy-halohexose, deoxyhalopentose, deoxy-aminopentose, deoxy-aminohexose, tetrose, heterosugar, carboxysugar, a derivative of the aforementioned sugars, a disaccharide derived from at least one of the aforementioned sugars, or a polysaccharide derived from at least one of the aforementioned sugars. Suitable sugars include, e.g., L-ribose, D-ribose, L-fucose, D-fucose, 2-deoxy-D-galactose, 3-deoxy-D-glucose, 6-deoxy-D-glucose, 2-deoxy-2-fluoro-D-glucose, 6-deoxy6-fluoro-D-glucose, L-lyxose, D-lyxose, L-rhamnose, L-allose, D-allose, L-altrose, D-altrose, L-galactose, D-galactose, L-xylose, D-xylose, D-gulose, L-mannose, D-mannose, L-idose, D-idose, Lmycarose, 6-keto-D-galactose, L-arabinose, D-arabinose, N-acetyl-D-galactosaminose, melibiose, lactose, maltose, D-galacturonose, L-talose, D-talose, 6-deoxy-6-azo-D-mannose, L-glucose, D-glucose, and mixtures thereof.

In some embodiments, the drug is a cardiac glycoside. For example and without limitation, the cardiac glycoside may be a scillarenin derivative, a bufalin derivative, a digitoxigenin derivative, or an ouabagenin derivative. In some embodiments, the drug is CEN09-106. In some embodiments, the drug is CEN09-107. In some embodiments, the drug is CEN-317. In some embodiments, the drug is CEN-319.

V. Linkers

In an EDC of the present disclosure (e.g., an EDC9 conjugate), the drug (e.g., cardiac glycoside or cardiotonic steroid) is coupled to the targeting moiety portion via a stable linker. The linker is long (generally about 60 Angstroms to about 150 Angstroms, for example at least about 50 Angstroms in length and more typically 100 Angstroms in length, or as long as about 200 Angstroms or even 300 Angstroms in length or longer), flexible and extendable, and in some embodiments positively charged, including by the presence of one or more heteroatoms (e.g., nitrogen) in an alkyl chain that forms all or part of the linker portion and is attached to the drug. In one important aspect, the present disclosure provides new linkers for linking anti-CD20 antibodies to cardiotonic steroids, cardiac glycosides, and other drugs that target the Na,K-ATPase according to the claims, and compositions and compounds in purified form useful in their synthesis, methods for which are also provided by the present disclosure. Coupling of a linker (e.g., as a completed linker portion) or formed step-wise, for example by coupling a targeting moiety bound to a first portion of the linker and then with either further portions of the linker or directly to a drug portion, which may optionally be in a synthetic intermediate in which the drug is bound to a second portion of the linker.

The linkers employed in the EDCs of the present disclosure are stable. For example, after administration, the EDC is stable and remains intact, e.g. the targeting moiety remains linked to the drug via the linker. The linkers are stable outside the target cell and remain uncleaved for efficacy. An effective linker will: (i) maintain the specific binding properties of the antibody; (ii) allow delivery of the conjugate or drug; (iii) remain stable and intact, e.g. not cleaved, for as long as the antibody and/or drug remains stable and intact; and (iv) maintain a cytotoxic, cell-killing effect or a cytostatic effect of the drug while the EDC is intact. By way of example, stable linkers are those that, when in an EDC of the present disclosure, show minimal (e.g., less than 10%) cleavage while present in the circulatory structure, at the surface of target tissue, at the surface of target cell, or in the extracellular matrix for a period of at least 4 to 8 hours or longer, such as 8 to 24 hours, or 1 to 10 days or longer; non-cleavable linkers are stable in these conditions for longer periods, including periods as long as 20 days or longer (Durcy, L. et. al. Bioconjugate Chem. 2010, 21, 5-13).

The length of a suitable linker may be determined by experimental measurements, for example by testing multiple linker lengths in the assay used to determine activity of the resulting EDC of the present disclosure. For example, if linker length is too short (not allowing the drug and targeting moiety to reach their binding sites simultaneously), one can readily identify and correct the problem to provide an EDC of the present disclosure. Typically, the linker length will be in the range of about 50 to about 300 Angstroms, or about 50 to about 200 Angstroms, more preferably about 60 to about 150 Angstroms. The linker length and composition is selected to ensure that the EDC remains stable in the circulatory structure where enzymes and other environmental substances may otherwise break it down and to reflect the distance from where the targeting moiety binds to its antigen and where the drug acts on its target. A wide variety of linkers that comply with these requirements are available or can be synthesized, which creates a very large class of EDC9 conjugates provided by the present disclosure, particularly when one considers the wide variety of linkers, therapeutic agents and targeting moieties that can be employed in the EDC of the present disclosure.

The linker, if conceptualized as a discrete entity instead of part of an EDC, is a monofunctional or multifunctional moiety that can be used to link one or more drugs to an antibody to form an EDC. EDC can be conveniently prepared using a linker having reactive functionality for binding to the drug and to the antibody. For example, a cysteine thiol, or an amine, e.g. a sulfur atom of a reduced disulfide bond, or an N-terminus or amino acid side chain such as lysine or histidine, of an antibody can form a bond with a functional group of a linker reagent or drug-linker reagent (composed of the drug and linker).

In some embodiments, the linker includes one or more ethylene glycol units (e.g., a polyethylene glycol). Without wishing to be bound by theory, it is believed that the presence of ethylene glycol units in the linker imparts flexibility, tunable spacing between the drug and the targeting moiety, and desirable solubility characteristics to the EDCs. In some embodiments, the linker includes the ethylene glycol unit(s) in close proximity to the targeting moiety, for example bound directly to the targeting moiety or bound to the targeting moiety by a short alkyl chain (e.g., a functionalized alkyl chain

having from 2 to about 20 atoms). In some embodiments, the linker includes a polyethylene glycol subunit having from about 6 to about 60 ethylene glycol repeat units, for example about 6, about 12, about 24, about 36, about 48, or about 60 ethylene glycol repeat units. In some preferred embodiments, the linker includes 24 ethylene glycol repeat units (also referred to herein as "PEG24"). In other preferred embodiments, the linker includes 36 ethylene glycol units ("PEG36").

In some embodiments, the linker includes a second portion which includes at least one heteroatom (e.g., nitrogen). Without wishing to be bound by theory, it is believed that the presence of the heteroatom(s) such as nitrogen in the linker enhances the solubility properties of the EDCs at physiological pH. Accordingly, the optimum linker, including the optimum number of heteroatoms included therein, may vary for different combinations of drug portions and targeting moiety portions.

In some embodiments, the heteroatom(s) are included in an alkyl chain (e.g., a functionalized heteroalkyl chain). The heteroalkyl chain may be bound to the drug at any suitable location such that the activity of the drug is not substantially diminished (e.g., through the C3 carbon when the drug is a cardiotonic steroid). In some embodiments, the heteroalkyl chain is bound to the targeting moiety directly, but in preferred embodiments the heteroalkyl chain is bound to the targeting moiety by one or more ethylene glycol units (e.g., a polyethylene glycol such as PEG24). In some embodiments, the linker includes a least one glycoside residue (e.g., an aminoglycoside), which is typically attached to the drug of the EDC.

In some embodiments, linkers of the present disclosure have a formula consistent with that of Formula (IV):

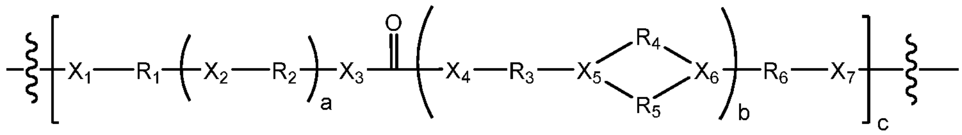

Formula (IV)

wherein:

$X_1$ is optionally present and, when present, may include (before coupling with the targeting moiety) a group reactive with a portion of the targeting moiety. For example and without limitation, when the targeting moiety is an antibody, antibody fragment, protein or peptide, the linker (before coupling with the targeting moiety) may include an electrophilic group, such as a maleimide, to enable coupling with the targeting moiety through a nucleophilic atom, such as a sulfur atom from a reduced disulfide bond, or a nitrogen atom from a lysine or histidine residue. Thus, in some embodiments, $X_1$ of Formula (IV) may be

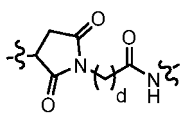

wherein d is 0 to 6;
each of $X_2$, $X_3$ and $X_4$ may optionally be present and may individually be selected from alkyl, ketone, -C(O)NH-, -C(O)NR$_8$-, -O-, -S-, -NH-, -NR$_9$-, wherein $R_8$ and $R_9$ are individually selected from alkyl (e.g., methyl), heteroalkyl, aryl, and heteroaryl;
$X_5$ and $X_6$ are each individually selected from CR$_{10}$ and N, wherein $R_{10}$ is H, branched alkyl, unbranched alkyl, saturated alkyl, or unsaturated alkyl;
$X_7$ is optionally present and may be selected from -C(O)-, -OC(O)-, -NHC(O)-, -NR$_{11}$C(O)-, wherein $R_{11}$ is H, branched alkyl, unbranched alkyl, saturated alkyl, or unsaturated alkyl;
$R_1$ is optionally present and may be selected from branched alkyl, unbranched alkyl, saturated alkyl, or unsaturated alkyl;
each of $R_2$, $R_3$ and $R_6$ may optionally be present and may individually be selected from branched alkyl, unbranched alkyl, saturated alkyl, and unsaturated alkyl;
each of $R_4$ and $R_5$ are optionally present and may be selected from branched alkyl, unbranched alkyl, saturated alkyl, or unsaturated alkyl, with the proviso that at least one of $R_4$ and $R_5$ must be present;
a is 0 to 99;
b is 0 to 99; and
c is 0 to 99.

In some preferred embodiments, the linker has a formula of Formula (II) wherein $X_1$ is

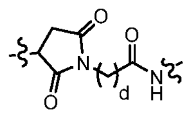

and d is 2; $X_2$ is -O-; $X_3$ is null; $X_4$ is -NH-; $X_5$ and $X_6$ are each N; $X_7$ is -NHC(O)-; $R_1$ is $-CH_2CH_2-$; $R_2$ is $-CH_2CH_2-$; $R_3$ and $R_6$ are each $-CH_2CH_2CH_2-$; $R_4$ and $R_5$ are each $-CH_2CH_2-$; a is 1-50; b is 1-10; and c is 1-10.

In one particularly preferred embodiment, the linker has a formula of Formula (II) wherein: $X_1$ is

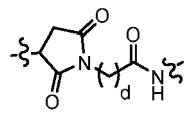

and d is 2; $X_2$ is -O-; $X_3$ is null; $X_4$ is -NH-; $X_5$ and $X_6$ are each N; $X_7$ is -NHC(O)-; $R_1$ is $-CH_2CH_2-$; $R_2$ is $-CH_2CH_2-$; $R_3$ and $R_6$ are each $-CH_2CH_2CH_2-$; $R_4$ and $R_5$ are each $-CH_2CH_2-$; a is 24 or 36; b is 1; and c is 1.

In another particularly preferred embodiment, the linker has a formula of Formula (II) wherein: $X_1$ is

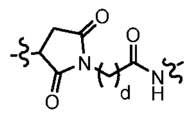

and d is 2; $X_2$ is -O-; $X_3$ is null; $X_4$ is -NH-; $X_5$ is $-N(CH_3)-$; $X_6$ is null; $X_7$ is -NHC(O)-; $R_1$ is $-CH_2CH_2-$; $R_2$ is $-CH_2CH_2-$; $R_3$ is $-CH_2CH_2CH_2-$; $R_4$ is null; $R_5$ and $R_6$, taken together, are $-CH_2CH_2CH_2-$; a is 24 or 36; b is 1; and c is 1.

In another particularly preferred embodiment, the linker has a formula of Formula (II) wherein: $X_1$ is

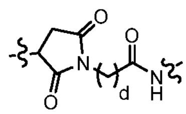

and d is 2; $X_2$ is -O-; $X_3$ is null; $X_4$ and $X_5$ are each -NH-; $X_6$ is null; $N_7$ is -NHC(O)-; $R_1$ is $-CH_2CH_2-$; $R_2$ is $-CH_2CH_2-$; $R_3$ is $-CH_2CH_2CH_2-$; $R_4$ is null; $R_5$ and $R_6$, taken together, are $-CH_2CH_2CH_2-$; a is 24 or 36; b is 1; and c is 1.

Accordingly, in some preferred embodiments of the present disclosure, the linker has a length of about 60 to about 150 Angstroms and comprises a polyethylene glycol portion including 24 or 36 ethylene glycol repeat units and a second portion including one or two amine nitrogen heteroatoms. Such linkers preferably include a first functional group (e.g., a maleimide moiety) for covalently binding the polyethylene glycol portion of the linker to a targeting moiety, and a second functional group (e.g., an amine) for covalently binding the second portion of the linker to the drug portion (e.g., by coupling to an activated form of the agent portion). In some embodiments, the second portion of the linker includes one amine nitrogen heteroatom. In other embodiments, the second portion of the linker includes two amine nitrogen heteroatoms.

By way of example to further describe suitable linkers, an EDC9 conjugate of the present disclosure may include a cardiac glycoside as the Drug, and a Linker comprising a heteroalkyl chain covalently attached to the glycosidic portion of the cardiac glycoside to provide a protonatable nitrogen heteroatom within about 10 atomic bonds of the C3 oxygen atom of the cardiac glycoside. For example, the CEN10-110 structure shown in Figure 1C includes a cardiac glycoside having a sugar moiety covalently bound to the C3 oxygen atom of the scillarenin steroidal moiety, and an amine functional group within about 10 atomic bonds (i.e., 8 atomic bonds) of the C3 oxygen atom of the scillarenin moiety:

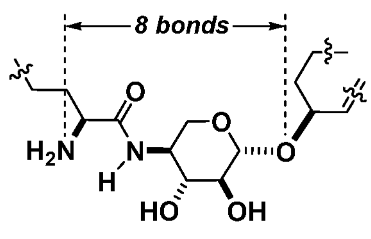

A complete "Drug-Linker" intermediate corresponding to the above example is shown in Figure 1D as "CEN10-110."

As another example to further describe suitable linkers, an EDC9 conjugate of the present disclosure may include a cardiotonic steroid as the Drug, and a Linker comprising a heteroalkyl chain covalently attached directly to the C3 "X" atom of the cardiotonic steroid to provide a protonatable nitrogen heteroatom within about 10 atomic bonds of the C3 "X" atom. For example, the CEN-371 structure shown in Figure 1C includes a piperizine-carbamate moiety bound directly to the 3C oxygen atom of bufalin (the Drug). The piperizine nitrogen closest to the bufalin moiety is within 10 atomic bonds (i.e., within 6 atomic bonds) of bufalin's C3 oxygen atom:

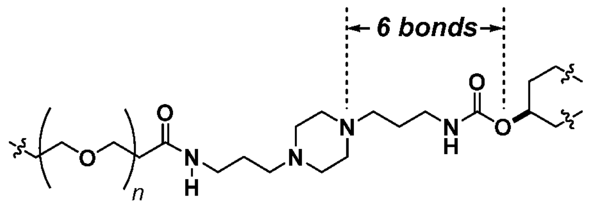


This CEN-371 exanmple also includes a second nitrogen heteroatom within 10 atomic bonds of bufalin's C3 oxygen substituent:

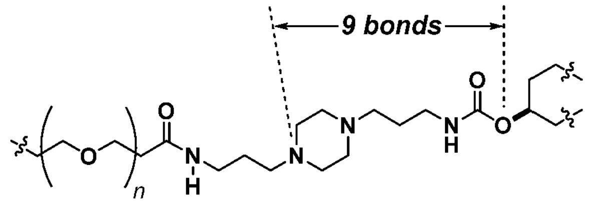


A complete "Drug-Linker" intermediate corresponding to the above example is shown in Figure 1D as "CEN-371."

Accordingly, in some embodiments, an EDC9 conjugate includes at least one nitrogen heteroatom (e.g., two nitrogen heteroatoms, three nitrogen heteroatoms, four nitrogen heteroatoms, etc.) within about 10 atomic bonds of the drug moiety. The nitrogen heteroatom(s) may each individually be primary, secondary, or tertiary in their neutral charge states.

Without wishing to be bound by theory, potency of this subclass of EDC9 conjugates may be due in part to inclusion of a nitrogen heteroatom in close proximity to the "X" atom attached to the C3 carbon of the Drug (Formulas (II)-(III)). At physiological pH, this nitrogen heteroatom may carry a net positive charge, which may enhance binding of the drug moiety to the extracellular active site of the alpha 1 subunit of Na,K-ATPase. This subunit of Na,K-ATPase is known to include an evolutionarily-conserved group of negatively charged amino acid residues (see, e.g., Laursen et al., PNAS, vol. 112(6), pages 1755-60 (2015)).

VI. Synthesis of EDC9 Conjugates

EDCs of the present disclosure and various intermediates useful in their synthesis provided by the invention are shown in Figures 1A-1G. In this figure, the structures are arranged as follows: Figure 1A shows illustrative cardiotonic steroids in their aglycone form; Figure 1B shows aminoglycosides of illustrative cardiotonic steroids in free amine form (not linked to linker), many of which are cardiac glycosides; Figure 1C shows illustrative drug moieties covalently attached to heteroatomic portions (e.g., heteroalkyl chains) of select Linkers described in the previous section. In Figure 1C, the heteroatomic portions of the Linker are depicted in free amine form. However, under physiological pH, at least one nitrogen atom of each heteroatomic Linker portion conveys a net positive charge within 6 to 10 atomic bonds of the C3 "X" atom of the Drug moiety [of Formula (II) or Formula (III)]; Figure 1D shows illustrative Drug-Linker compounds ready for coupling to reduced thiols of an antibody or antibody fragment via maleimide coupling; Figure 1E shows an illustrative Drug-Linker compound ready for coupling to an antibody or antibody fragment via aldehyde coupling; Figure 1F shows an illustrative example of an EDC of Formula (I) with the [TARGETING MOIETY]-[LINKER]-[DRUG] being an [antibody]-[PEG36]-[CEN09-106], respectively - the conjugation achieved through reaction of CEN10-131 to reduced cysteine thiols. Although Figure 1F shows a single antibody coupled with a single Drug-Linker (i.e., n=1), more than one Drug-Linker may be covalently linked to the antibody through other reduced cysteine thiol(s) (e.g., *n* may be greater than 1, such as 1 to about 10, about 4 to about 8, or about 4 to about 6); Figure 1G shows an illustrative example of an EDC of Formula (I) with the [TARGETING MOIETY]-[LINKER]-[DRUG] being an [antibody]-[PEG36]-[CEN09-106], respectively-

the conjugation achieved through reaction of CEN-332 to aldehyde groups produced by oxidization of N-linked glycans on the Fc region in the $CH_2$ domain. Although Figure 1G shows a single antibody coupled with a single Drug-Linker (i.e., n=1), more than one Drug-Linker may be covalently linked to the antibody through other aldehyde group(s) produced by oxidation of N-linked glycans on the Fc region in the $CH_2$ domain (e.g., *n* may be greater than 1, such as 1 to about 10, about 4 to about 8, or about 4 to about 6).

VII. Methods of Treatment

The references to methods of treatment are to be interpreted as references to the compounds, pharmaceutical compositions and medicaments of the present invention for use in a method for treatment of the human (or animal) body by therapy.

Though these EDC9 could be used to treat any disease where one strives to deplete cells expressing CD20 such as cancer, Lupus or multiple sclerosis. One very important aspect of the present disclosure provides EDCs useful in the treatment of NHL. These EDCs are characterized in that they are comprised of an antibody or binding fragment thereof that binds CD20, rituximab or its variable domain binding fragment being a suitable example of such an antibody, and is linked by a non-cleavable linker, PEG being a suitable example of such a linker, and a drug, CEN09-106 being one suitable example of such a drug. The present disclosure included methods, and medicaments useful in such methods, in which a patient with NHL is treated by administration of an EDC of the invention by IV infusion over repeated cycles including treatments regimens of once weekly, twice monthly, once every three weeks, and once a month. An EDC may be administered by IV infusion to a patient in need of treatment for a condition for which rituximab or other CD20 targeting moiety is approved for treatment at a frequency (and, optionally, molar dose ratio) that is identical to that approved for the targeting moiety alone for such indication.

Dosing of EDCs made and used in accordance with the present disclosure may vary according to the physician, patient, disease, and commercial availability of various formulations of the drug, but the EDC may be administered by intravenous infusion to provide a single therapeutically effective dose of about 0.1 mg/kg to about 10 mg/kg, such as about 0.5 mg of EDC to about 5 mg of EDC per kg of patient weight, e.g. an adult 70 kg human would get a dose between about 100 mg (140 for the ideal) to about 500 mg (350 for the ideal) with each administration of the drug. For many, if not most, of the diseases treatable, most patients will receive more than one administration of the drug, as per the preceding paragraph. Other doses and dose frequencies may be used, however, and where the targeting moiety is itself an approved drug, the EDC will be given on an equivalent molar basis or less, given the EDC's greater potency and specificity. For example the EDC may be administered on a molar basis that is EDC Dose: Targeting Moiety Alone Dose of 1:1 to 1:5 or event 1:10.

Among the diseases treatable with EDCs of the invention at these doses, diseases of particular note include low-grade or follicular CD20-positive NHL (single-agent therapy), optionally in patients whose disease recurred or did not respond to initial treatment; follicular CD20-positive NHL, optionally as first line chemotherapy or as a single-agent follow-up therapy; low-grade or follicular CD20-positive NHL as a single-agent follow-up therapy for patients who responded to initial treatment with CVP chemotherapy; CD20-positive diffuse large B-cell NHL, optionally as an initial treatment in combination with CHOP chemotherapy; CD20-positive chronic lymphocytic leukemia in combination with FC chemotherapy as an initial treatment or as a treatment after disease has recurred; rheumatoid arthritis with methotrexate, to reduce the signs and symptoms of moderate to severe active RA in adults, optionally after treatment with a tumor necrosis factor (TNF) antagonist fails or other treatment failure; and granulomatosis with polyangiitis (GPA) (Wegener's Granulomatosis) and microscopic polyangiitis (MPA) with glucocorticoids. Given that most patients with double hit lymphomas (DHL) will die rapidly from DHL, development of new and effective agents is a significant unmet medical need. DHL represent a subset of highly aggressive B-cell malignancies characterized by the presence of recurrent cytogenetic rearrangements affecting MYC and BCL2 and/or BCL6. Therefore drugs for MYC-driven lymphomas is a significant unmet medical need and drugs such as the ones used as the drug-portion of the EDC9 conjugates disclosed herein have been shown to inhibit c-myc expression.

Optionally, an EDC9 conjugate as disclosed herein may be administered with a BCL2 inhibitor, such as ABT-199. An EDC9 conjugate as disclosed herein may be administered with a TNF-related apoptosis-inducing ligand (TRAIL). An EDC9 conjugate as disclosed herein may be administered with an FGFR tyrosine kinase inhibitor. Alternatively, an EDC9 conjugate as disclosed herein may be administered with an mTOR inhibitor. An EDC9 conjugate as disclosed herein may be administered with a Rho kinase (ROCK) inhibitor. Or, an EDC9 conjugate as disclosed herein may be administered with gemcitabine.

A method of treating cancer or an inflammatory disease in a subject may comprise administering an EDC9 conjugate as disclosed herein to the subject.

A method of treating a lymphoma in a subject may comprise administering an EDC9 conjugate as disclosed herein to the subject. The EDC9 conjugate may include a targeting moiety comprising rituximab, a drug portion comprising scillarenin and an aminoglycoside covalently bound to the C3 oxygen substituent of scillarenin, and a linker portion

comprising a polyethylene glycol spacer including 36 PEG units. The EDC9 conjugate may be Rituximab-CEN10-131. The targeting moiety may comprise rituximab, the drug portion may comprise bufalin and a piperazine-carbamate moiety covalently bound to the C3 oxygen substituent of bufalin, and the linker portion may comprise a polyethylene glycol spacer including 24 PEG units. Or, the EDC9 conjugate may be Rituximab-CEN-375. The EDC9 conjugate used in this method may have an antibody loading (i.e., *n* of Formula (I)) of about 1 to about 10, about 4 to about 8, or about 4 to about 6.

A method of treating a non-Hodgkin's lymphoma in a subject may comprise administering an EDC9 conjugate as disclosed herein to the subject. The EDC9 conjugate may include a targeting moiety comprising rituximab, a drug portion comprising scillarenin and an aminoglycoside covalently bound to the C3 oxygen substituent of scillarenin, and a linker portion comprising a polyethylene glycol spacer including 36 PEG units. The EDC9 conjugate may be Rituximab-CEN10-131. The targeting moiety may comprise rituximab, the drug portion may comprise bufalin and a piperazine-carbamate moiety covalently bound to the C3 oxygen substituent of bufalin, and the linker portion may comprise a polyethylene glycol spacer including 24 PEG units. Or, the EDC9 conjugate may be Rituximab-CEN-375. The EDC9 conjugate used in this method may have an antibody loading (i.e., *n* of Formula (I)) of about 1 to about 10, about 4 to about 8, or about 4 to about 6.

A method of treating a leukemia in a subject may comprise administering an EDC9 conjugate as disclosed herein to the subject. The EDC9 conjugate may include a targeting moiety comprising rituximab, a drug portion comprising scillarenin and an aminoglycoside covalently bound to the C3 oxygen substituent of scillarenin, and a linker portion comprising a polyethylene glycol spacer including 36 PEG units. The EDC9 conjugate may be Rituximab-CEN10-131. The targeting moiety may comprise rituximab, the drug portion may comprise bufalin and a piperazine-carbamate moiety covalently bound to the C3 oxygen substituent of bufalin, and the linker portion may comprise a polyethylene glycol spacer including 24 PEG units. The EDC9 conjugate may be Rituximab-CEN-375. The EDC9 conjugate used in this method may have an antibody loading (i.e., *n* of Formula (I)) of about 1 to about 10, about 4 to about 8, or about 4 to about 6.

A method of treating a B-cell malignancy in a subject may comprise administering an EDC9 conjugate as disclosed herein to the subject. The EDC9 conjugate may include a targeting moiety comprising rituximab, a drug portion comprising scillarenin and an aminoglycoside covalently bound to the C3 oxygen substituent of scillarenin, and a linker portion comprising a polyethylene glycol spacer including 36 PEG units. The EDC9 conjugate may be Rituximab-CEN10-131. The targeting moiety may comprise rituximab, the drug portion may comprise bufalin and a piperazine-carbamate moiety covalently bound to the C3 oxygen substituent of bufalin, and the linker portion may comprise a polyethylene glycol spacer including 24 PEG units. The EDC9 conjugate may be Rituximab-CEN-375. The EDC9 conjugate used in this method may have an antibody loading (i.e., *n* of Formula (I)) of about 1 to about 10, about 4 to about 8, or about 4 to about 6.

A method of treating a lymphoma in a subject may comprising administering an EDC9 conjugate as disclosed herein to the subject. The EDC9 conjugate may include a targeting moiety comprising a rituximab biosimilar, a drug portion comprising scillarenin and an aminoglycoside covalently bound to the C3 oxygen substituent of scillarenin, and a linker portion comprising a polyethylene glycol spacer including 36 PEG units. The EDC9 conjugate may comprise CEN10-131. The targeting moiety may comprise a rituximab biosimilar, the drug portion may comprise bufalin and a piperazine-carbamate moiety covalently bound to the C3 oxygen substituent of bufalin, and the linker portion may comprise a polyethylene glycol spacer including 24 PEG units. The EDC9 conjugate may comprise CEN-375. The EDC9 conjugate used in this method may have an antibody loading (i.e., *n* of Formula (I)) of about 1 to about 10, about 4 to about 8, or about 4 to about 6.

A method of treating a non-Hodgkin's lymphoma in a subject may comprise administering an EDC9 conjugate as disclosed herein to the subject. The EDC9 conjugate may include a targeting moiety comprising a rituximab biosimilar, a drug portion comprising scillarenin and an aminoglycoside covalently bound to the C3 oxygen substituent of scillarenin, and a linker portion comprising a polyethylene glycol spacer including 36 PEG units. The EDC9 conjugate may comprise CEN10-131. The targeting moiety may comprise a rituximab biosimilar, the drug portion may comprise bufalin and a piperazine-carbamate moiety covalently bound to the C3 oxygen substituent of bufalin, and the linker portion may comprise a polyethylene glycol spacer including 24 PEG units. The EDC9 conjugate may comprise CEN-375. The EDC9 conjugate used in this method may have an antibody loading (i.e., *n* of Formula (I)) of about 1 to about 10, about 4 to about 8, or about 4 to about 6.

A method of treating a leukemia in a subject may comprise administering an EDC9 conjugate as disclosed herein to the subject. The EDC9 conjugate may include a targeting moiety comprising a rituximab biosimilar, a drug portion comprising scillarenin and an aminoglycoside covalently bound to the C3 oxygen substituent of scillarenin, and a linker portion comprising a polyethylene glycol spacer including 36 PEG units. The EDC9 conjugate may comprise CEN10-131. The targeting moiety may comprise a rituximab biosimilar, the drug portion may comprise bufalin and a piperazine-carbamate moiety covalently bound to the C3 oxygen substituent of bufalin, and the linker portion may comprise a polyethylene glycol spacer including 24 PEG units. The EDC9 conjugate may comprise CEN-375. The EDC9 conjugate used in this method may have an antibody loading (i.e., *n* of Formula (I)) of about 1 to about 10, about 4 to about 8, or about 4 to about 6.

A method of treating a B-cell malignancy in a subject may comprise administering an EDC9 conjugate as disclosed herein to the subject. The EDC9 conjugate may include a targeting moiety comprising a rituximab biosimilar, a drug portion comprising scillarenin and an aminoglycoside covalently bound to the C3 oxygen substituent of scillarenin, and a linker portion comprising a polyethylene glycol spacer including 36 PEG units. The EDC9 conjugate may comprise CEN10-131. The targeting moiety may comprise a rituximab biosimilar, the drug portion may comprise bufalin and a piperazine-carbamate moiety covalently bound to the C3 oxygen substituent of bufalin, and the linker portion may comprise a polyethylene glycol spacer including 24 PEG units. The EDC9 conjugate may comprise CEN-375. The EDC9 conjugate used in this method may have an antibody loading (i.e., *n* of Formula (I)) of about 1 to about 10, about 4 to about 8, or about 4 to about 6.

A method of treating a cancer in a subject may comprise administering Rituximab-CEN10-131 to the subject. The cancer may be a CD20-positive cancer. The cancer may be a lymphoma. The cancer may be a non-Hodgkin's lymphoma. The cancer may be a leukemia. The cancer may be a B-cell malignancy. The Rituximab-CEN10-131 EDC9 conjugate used in this method may have an antibody loading (i.e., *n* of Formula (I)) of about 1 to about 10, about 4 to about 8, or about 4 to about 6.

A method of treating a cancer in a subject may comprise administering Rituximab-CEN-375 to the subject. The cancer may be a CD20-positive cancer. The cancer may be a lymphoma. The cancer may be a non-Hodgkin's lymphoma. The cancer may be a leukemia. The cancer may be a B-cell malignancy. The Rituximab-CEN-375 EDC9 conjugate used in this method may have an antibody loading (i.e., *n* of Formula (I)) of about 1 to about 10, about 4 to about 8, or about 4 to about 6.

A method of treating a cancer in a subject may comprise administering anti-CD20(2H7)-CEN10-131 to the subject.

The cancer may be a CD20-positive cancer. The cancer may be a lymphoma. The cancer may be a non-Hodgkin's lymphoma. The cancer may be a leukemia. The cancer may be a B-cell malignancy. The anti-CD20(2H7)-CEN10-131 EDC9 conjugate used in this method may have an antibody loading (i.e., *n* of Formula (I)) of about 1 to about 10, about 4 to about 8, or about 4 to about 6.

A method of treating a cancer in a subject may comprise administering anti-CD20(2H7)-CEN-375 to the subject. The cancer may be a CD20-positive cancer. The cancer may be a lymphoma. The cancer may be a non-Hodgkin's lymphoma. The cancer may be a leukemia. The cancer may be a B-cell malignancy. The anti-CD20(2H7)-CEN10-131 EDC9 conjugate used in this method may have an antibody loading (i.e., *n* of Formula (I)) of about 1 to about 10, about 4 to about 8, or about 4 to about 6.

In any of the foregoing methods, the EDC9 conjugate may be administered in an injectable form. The EDC9 conjugate may be administered about once per week to about once every 4 weeks, about once per week, about once every 2 weeks, about once every 3 weeks, or about once every 4 weeks. The EDC9 conjugate may be administered at a dose of about 0.1 mg per kilogram of the subject's body weight ("mg/kg") to about 10 mg/kg, for example about 0.5 mg/kg to about 5 mg/kg, about 0.1 mg/kg, about 0.2 mg/kg, about 0.3 mg/kg, about 0.4 mg/kg, about 0.5 mg/kg, about 0.6 mg/kg, about 0.7 mg/kg, about 0.8 mg/kg, about 0.9 mg/kg, about 1 mg/kg, about 1.5 mg/kg, about 2 mg/kg, about 2.5 mg/kg, about 3 mg/kg, about 3.5 mg/kg, about 4 mg/kg, about 4.5 mg/kg, about 5 mg/kg, about 5.5 mg/kg, about 6 mg/kg, about 6.5 mg/kg, about 7 mg/kg, about 7.5 mg/kg, about 8 mg/kg, about 8.5 mg/kg, about 9 mg/kg, about 9.5 mg/kg, about 10 mg/kg.

A method of treating a subject on Rituximab therapy may comprise identifying the subject as receiving Rituximab therapy and no longer responding to the Rituximab therapy, discontinuing the Rituximab therapy, and administering an EDC9 conjugate as disclosed herein to the subject. The step of administering the EDC9 conjugate may commence about 1 week, about 2 weeks, about 3 weeks, or about 4 weeks after the step of discontinuing the Rituximab therapy. The EDC9 conjugate may comprise a targeting moiety comprising rituximab, a drug portion comprising scillarenin and an aminoglycoside covalently bound to the C3 oxygen substituent of scillarenin, and a linker portion comprising a polyethylene glycol spacer including 36 PEG units. The EDC9 conjugate may comprise CEN10-131. The EDC9 conjugate may be Rituximab-CEN10-131.

The EDC9 conjugate may comprise a targeting moiety comprising rituximab, a drug portion comprising bufalin and a piperazine-carbamate moiety covalently bound to the C3 oxygen substituent of bufalin, and a linker portion comprising a polyethylene glycol spacer including 24 PEG units. The EDC9 conjugate may comprise CEN-375. The EDC9 conjugate may be Rituximab-CEN-375. The step of administering the EDC9 conjugate to the subject may comprise administering the EDC9 conjugate to the subject at a dose and dose frequency similar to or the same as the Rituximab therapy. The subject in these methods may have a cancer selected from the group consisting of: a lymphoma, a non-Hodgkin's lymphoma, a leukemia, a B-cell malignancy, and non-Hodgkin's lymphoma.

EXAMPLES

As described in the following examples, EDCs of Formula (I) were synthesized (Example 1) and tested in

various in vitro and in vivo studies with results that demonstrate they are new anti-cancer agents that should be further developed and tested for potential use in human therapies for any disease currently or later found to be treatable with anti-CD20 antibody therapies.

**Example 1. Synthesis of EDCs; Methods for Inhibition of Cell Proliferation (Tumor Cell Growth or Tumor Growth) and Cytotoxicity; Xenograft Study Methodology**

Targeting moieties: The EDCs used in the studies described in these examples utilized antibody Targeting Moieties. Rituximab (Genentech; South San Francisco, CA) is a chimeric monoclonal antibody against CD20 that was originally developed by IDEC Pharmaceuticals under the name IDEC-C2B8, and anti-CD20 (2H7) is a mouse monoclonal IgG2b kappa antibody that binds human CD20 and is secreted/produced by a B-cell hybridoma cell line called Clone 2H7 (Biolegend; Cat#302302). Human IgG from normal serum (and thus "non-targeted" for purposes of this study) (purchased from Innovative Research; Cat#IR-HU-GF) was conjugated to prepare control EDC.

Synthesis of EDCs: EDCs of the present disclosure can be synthesized via conjugation chemistry as follows. For these studies, the antibody (rituximab or anti-CD20 (2H7) murine monoclonal or other control or test antibody) and a maleimide activated drug linker moiety CEN10-131 (or CEN-375) were used to make EDC9 conjugates of the present disclosure (or a control EDC that does not bind to CD20). In the EDC9 conjugates used in these examples, the targeting moiety is a monoclonal antibody that binds to CD20, and the drug (a cardiac glycoside in these examples) is a scillarenin derivative (CEN09-106) or a bufalin derivative (CEN-371), and the linker is either PEG24 or PEG36 modified for maleimide activated coupling (e.g. MAL-PEG24 or MAL-PEG36). In brief, the EDCs were prepared by conjugation chemistry involving: (1) reduction of antibody interchain disulfides, (2) addition of maleimide activated-PEG36-CEN09-106 (CEN10-131) or maleimide activated-PEG24-CEN-371 (CEN-375) and (3) ultra filtration (Amicon Ultra 30 kDa molecular weight cutoff) to remove uncoupled CEN10-131 or CEN-375.

First, antibody reduction was carried out at antibody concentrations between 1-10 mg/ml in a sodium acetate/citrate buffer (150 mM sodium acetate, 300 mM sodium citrate; pH 7.5). The antibodies were reduced in the presence of 0.43 μmol of diethylenetriamine pentaacetic acid (DTPA) (MP Biomedical LLC) per mg antibody and 4 molar equivalents (per molar equivalent of antibody) of tris(2-carboxyethyl)phosphine (TCEP) (cat. number: HR2-651, Hampton Research) and incubated at 37°C for 2 hours then transferred to room temperature. For conjugation, 10 equivalents of CEN10-131 or 12 equivalents of CEN-375 were added to the reduced antibody and reacted for 2 hours at room temperature, after which the EDCs were then separated from uncoupled CEN10-131 or CEN-375 by centrifugal concentration using Amicon μLtra 30,000 MWCO (Millipore, Billerica, MA) and buffer exchanged with phosphate buffered saline (PBS). The EDCs were then stored at 2-8°C in PBS at concentrations ranging from 1-10 mg/ml until used for the studies described here. Control EDC was prepared by conjugation of Human IgG purified from normal serum with CEN10-131 by the above methodology.

EDC loading (number of drugs per antibody) was determined by absorbance at 280 nm and 299 nm. First, the absorbance of free antibody was measured at 280 nm (A280Ab) and 299 nm (A299Ab) to determine antibody constant [Constant Ab]. Next, the absorbance of free drug was measured at 280 nm (A280drug) and 299 nm (A299drug) to determine drug constant [Constant Drug]. Finally, the absorbance of the EDC was measured at 280 nm (A280Conj) and 299 nm (A299Conj). The antibody molar extinction coefficient at 280 nm = 204,000 $M^{-1}cm^{-1}$. The drug molar extinction coefficient at 299 nm = 5623 $M^{-1}cm^{-1}$. The drug loading was determined to be approximately 4-8 drugs per 1 antibody, using the following approximation calculations:

$$[\text{Constant Ab}] = \text{A299Ab} / \text{A280Ab}$$

$$[\text{Constant Drug}] = \text{A299drug} / \text{A280drug}$$

$$\text{A280Ab*} = \text{A280Conj} - (\text{A299Conj} - [\text{Constant Ab}] \times \text{A280Conj}) / ([\text{Constant drug}] - [\text{Constant Ab}])$$

$$\text{A299drug*} = \text{A299} - [\text{Constant Ab}] \times \text{A280Ab}$$

$$\text{Antibody concentrations} = \text{A280Ab*} / 204{,}000\ M^{-1}cm^{-1}$$

$$\text{Drug concentration} = \text{A299drug*} / 5623\ M^{-1}cm^{-1}$$

$$\text{Drug loading} = \text{drug concentration} / \text{antibody concentration}$$

A299drug* = drug component of ADC

A280Ab* = antibody component of ADC

The resulting EDCs 'were subjected to hydrophobic interaction chromatography (HIC) analysis as follows. The EDCs were loaded on a HIC (POLYPROPYL A™ Column; 100 × 4.6mm; 3um; 1500 Å; Item 104PR0315; Ser# L0242F) and monitored over a timed gradient from 100% Buffer A (2M Ammonium Sulfate/50mM PBS, pH 7) to 100% Buffer B (50mM PBS, pH 7). The EDC monitoring results were compared to those for the corresponding antibody. Integration was performed at 215 nm and showed DAR values consistent with that found on UV-VIS (DAR ~4-8). The % unlabeled antibody was <5% in all cases. Methods for Inhibition of Cell Proliferation (Tumor Cell Growth or Tumor Growth) and Cytotoxicity: Cell proliferation and cytotoxicity assays were used to generate the results reported in these examples. The cytotoxic activity of the EDC of the invention (anti-CD20 conjugates) and various controls, often including the free anti-CD20 antibody rituximab were evaluated in vitro against two CD20 positive lymphoma cell lines from the ATCC. These cell lines are the Ramos (Human Burkitt's lymphoma) and SUDHL4 (Non-Hodgkin lymphoma) cell lines. Generally, in these assays, cells were first plated at a density of approximately 2000 per well of a 384-well white tissue culture treated microtiter plate in 20 μL complete media (RPMI1640 supplemented with 10% fetal bovine serum and 30 μg/mL gentamycin) and the plates incubated (the cells "grown") for 24 hours at 37°C in 5% $CO_2$ in a humidified incubator. Second, various amounts of test agent (e.g., EDC or rituximab) or other control were then added to the cells, which were then incubated for 72 hours. Cell viability was then analyzed using the CellTiter-Glo luminescent cell viability assay and standard package insert protocol of the manufacturer (Promega, Madison, WI). EC50 values of the test compounds for each cell line were determined using GraphPad Prism 5 software

Xenograft Study Methodology:

SU-DHL-4 cells (ATCC), grown in RPMI1640 supplemented with 10% fetal bovine serum and 30 μg/mL gentamycin at 37°C in 5% $CO_2$, were washed, suspended in Dulbecco's PBS (Hyclone), and inoculated subcutaneously ($1 \times 10^7$ cells suspended in 0.2 ml per mouse) into the flanks of female CB17SCID mice 36-42 days old (SU-DHL-4) (Charles River Laboratories). When mean tumor size reached the desired mean tumor size the animal was randomized into a treatment group.

Tables 1-2 are summary tables of experiments described in the following Examples and other data not described in detail herein, and are provided for the convenience:

Table 1. Cytotoxicities of Select Drugs and Drug-Linker Intermediates

| Drug/ Drug-Linker intermediate | EC50 in picoM (% viab.) | |
|---|---|---|
| | Ramos | SUDHL4 |
| CEN09-106 | 1,900 (0) | 2,070 (0) |
| CEN-319 | 5,800 (0) | 8,400 (ND) |
| CEN-371 | 7,805 (0) | 11,000 (0) |
| CEN-372 | 3,600 | 4,400 |
| CEN-373 | 4,000 | 5,900 |
| CEN-381 | >50,000 | >50,000 |
| Bufalin | 7,300 | 16,000 |
| Scillarenin | 17,000 | 18,000 |
| Digitoxigenin | >50,000 | >50,000 |

Table 2. Cytotoxicities of Select EDC9 conjugates vs. Rituximab

| Targeting Moiety | Linker-Agent Includes: | Loading (*n*) | EC50 in picoM (% viab.) | |
|---|---|---|---|---|
| | | | Ramos | SU-DHL-4 |
| Rituximab | none | n/a | 1231 (80) | 1342 (50) |
| Rituximab | PEG36, CEN09-106 ("CEN10-131") | 4-8 | 89 (0) | 37 (0) |
| Rituximab | PEG24, CEN-371 ("CEN-375") | 4-8 | 180 (0) | 126 (0) |
| Anti-CD20 | PEG36, CEN09-106 (CEN10-131) | 4-8 | 610 (0) | 139 (0) |

**Example 2. Potency of EDCs prepared from antibodies targeting human CD20**

When tested against the CD20 positive cell line Ramos, the EDC9 conjugates prepared from Rituximab (Rituximab-CEN10-131) and anti-CD20(2H7) antibody (anti-CD20(2H7)-CEN10-131) showed strong cytotoxic activity (0% viable cells after a 72 hour exposure) and $EC_{50}$ values of 89 pM and 610 pM respectively while the unconjugated Rituximab antibody (Rituximab) showed no direct cytotoxic activity (in the absence of human serum complement) when tested at concentrations from 10 pM to 200 nM (Figure 2). This result showed that the attachment of maleimide-PEG36-CEN09-106 (CEN10-131) to anti-CD20 antibodies confers in vitro cytotoxic activity against the CD20(+) B-cell lymphoma cell line Ramos even in the absence of serum complement (i.e not eliciting complement dependent cytotoxicity (CDC)). While not wishing to be bound by theory, these data may suggest that EDC9 conjugates of the present disclosure may elicit an immunogenic response as a result of cell necrosis (rather than apoptosis). When cells are exposed to excessive physical or chemical stresses, cells die by necrosis. Necrotic cells are phagocytosed or endocytosed by phagocytes. The plasma membrane integrity of necrotic cells is lost and cellular components are leaked into the extracellular space. Among the released cellular components, immune stimulatory molecules such as High-Mobility Group Box 1 Protein (HMGB1), uric acid, heat shock proteins (HSPs), nucleotides. These molecules activate phagocytes via receptor binding and induce the production of inflammatory cytokines.

**Example 3. Providing net positive charge in proximity of X [of Formula (II) or Formula (III)]**

Drug-Linker intermediate CEN-375 was constructed from a heterobifunctional amine-reactive PEG24-maleimide Linker and CEN-371. As shown in Figure 1C, CEN-371 is a bufalin derivative including a piperizine-carbamate attached to the C3 oxygen atom. The piperizine-carbamante moiety is therefore in the same location as a sugar moiety would be in an analogous cardiac glycoside. The primary amine group on the piperizine-carbamate moiety was coupled to the heterobifunctional amine-reactive PEG24-maleimide Linker to form the Drug-Linker intermediate CEN-375 (Figure 1D).

An EDC9 conjugate prepared from Rituximab conjugated to CEN-375 through reduced cysteine thiols (Rituximab-CEN-375) was tested against the CD20 positive cell line Ramos. The EDC9 conjugate showed strong cytotoxic activity (0% viable cells after a 72 hour exposure) and an EC50 value of 180 pM, while the unconjugated Rituximab antibody (Rituximab) showed mild cytotoxic activity (~80% viable cells after a 72 hour exposure) and an EC50 value of 1 nM (Figure 3). This result shows that EDC9 conjugates including a Drug that includes a piperizine-carbamate moiety in place of a sugar moiety provides similar cytotoxicity to other EDC9 conjugates comprising a cardiac glycoside as the Drug.

Without wishing to be bound by theory, the potency of this subclass of EDC9 conjugates may be due in part to inclusion of a nitrogen heteroatom in close proximity to the C3 "X" atom of the Drug (Formulas (II)-(III)). At physiological pH, this nitrogen heteroatom may carry a net positive charge, which may enhance binding of the Drug moiety to the extracellular active site of the alpha 1 subunit of Na,K-ATPase, which is known to include an evolutionarily-conserved group of negatively charged amino acid residues (see, e.g., Laursen et al., PNAS, vol. 112(6), pages 1755-60 (2015)).

**Example 4. Cytotoxicity of EDC9 conjugates against SU-DHL-4 cell line**

When tested against the CD20 positive cell line SUDHL4, the EDC9 conjugate Rituximab-CEN10-131 was strongly cytotoxic (0% viable cells after a 72 hour exposure) and displayed an $EC_{50}$ of approximately 37 pM, while the unconjugated Rituximab antibody showed mild cytotoxicity (~50% viable cells after a 72 hour exposure) and an $EC_{50}$ value of 1 nM (Figure 4). This result provides further evidence that the attachment of maleimide-PEG36-CEN09-106 (CEN10-131) to anti-CD20 antibodies confers in vitro cytotoxic activity against CD20(+) B-cell lymphoma cell lines (SUDHL4).

When tested against the CD20 positive cell line SUDHL4, the EDC9 conjugate Rituximab-CEN-375 was strongly cytotoxic (0% viable cells after a 72 hour exposure) and an EC50 of approximately 126 pM, while the unconjugated Rituximab antibody showed mild cytotoxicity (~50% viable cells after a 72 hour exposure) and an EC50 value of 1 nM (Figure 4). This result provides further evidence that, in the context of EDC9, piperizine-carbamate can replace the sugar moiety of cardiac glycosides (demonstrated against SUDHL4 in this case) without significant loss of potency.

Attachment of maleimide-PEG36-CEN09-106 (CEN10-131) to the anti-CD20 antibody Rituximab confers similar *in vitro* cytotoxic activity, relative to the unconjugated form of the anti-CD20 antibody Rituximab, against additional CD20(+) B-cell lymphoma cell lines: HL-60 [EC50=55 pM] an acute promyelocytic leukemia (ATCC), Raji [EC50 = 270 pM] a EBV positive Burkitt lymphoma (ATCC), Bjab [680 pM] a histiocytic lymphoma (DSMZ), and WSU-NHL [140 pM] a histiocytic lymphoma (DSMZ). These results show the potential of the EDC9 class of EDCs disclosed herein to treat a wide range of CD20 positive B-cell malignancies.

**Example 5. EDC9 toxicity: Peripheral blood mononuclear cell (PBMC) testing, preliminary maximum tolerated dosage in non-human primate *(Macaca fascicularis)***

PBMCs from healthy donors and human B-cell lymphoma cells (SUDHL4 and Ramos) were exposed to an EDC9 conjugate (Rituximab-CEN10-131) and analyzed for indications of cell viability post exposure. Results are diagramed in Figure 5. Briefly, PBMCs from healthy donors were immediately plated ($2 \times 10^5$/ml) in RPMI1640 supplemented with 10% fetal calf serum (FCS) and treated with EDC for 20 hours. Cells were then collected and stained with FITC-conjugated annexin-V (BioLegend) and propidium iodide (Invitrogen) for 15 minutes at room temperature. Apoptosis was measured using a FACSCanto flow cytometer (Becton-Dickinson) and evaluated with the FlowJo software. SUDHL4 and Ramos cell cytotoxity assays where performed as described in Example 1. The results of this study show the exposure to the EDC9 Rituximab-CEN10-131 is essentially non-toxic to the majority of human PBMCs at concentrations in which 100% of the Ramos and SUDHL4 cells are killed.

Preliminary data of a toxicology study in cynomolgus monkey. Two animals with implanted with Data Sciences International (DSI) telemetry implants to monitor and record Electrocardiographic (ECG) signals and blood pressure during and post infusion. Animals (weighing about 3 kg each) were infused with Rituximab-CEN10-131 at a dosage of 5 mg/kg over approximately 3 hours or at 10 mg/kg over 7 hrs. The animal that received 5 mg/kg displayed normal ECG and blood pressure measurements over the period of infusion out to 48 hours post infusion. The animal that received 10 mg/kg displayed normal ECG and blood pressure measurements over the period of infusion out to 15 hours post infusion when the animal developed atrioventricular block (AV block) consistent with cardiac glycoside poisoning. It is worth noting that at 5 mg/kg the level of antibody-bound cardiac glycoside in the serum approaches 2000 nM, which is about 200 times greater that the level of free cardiac glycoside that would be considered maximally tolerated (10 nM). Immunophenotyping performed on samples from the 5 mg/kg animal showed a marked decline in total B-cell count out to 14 days post exposure (similar to Rituiximab) and a transient depletion of CD3(-)/CD16(+) Natural Killer (NK) cells at 24 hours post exposure that rebounded at 48 hours post exposure and returned to baseline levels at 72 hours post exposure. Total T-cell (% CD3+), cytotoxic T-cell (CD3+CD8+), and helper T-cell (CD3+CD4+) remained within the range observed in predose samples. Preliminary studies of toxicity in the non-human primate (*Macaca fascicularis*) show a maximally tolerated dose of EDC9 (Rituximab-CEN10-131) of at least 5 mg/kg body weight.

**Example 6. Antitumor effects of EDC9 conjugates compared to Rituximab**

The antitumor effects of Rituximab-CEN10-131 were compared to Rituximab, the standard of care for human B-cell lymphoma. Mice bearing SUDHL4 tumors were administered single 5 mg/kg doses of either EDC-CD20 or Rituximab (Figure 6A). Rituximab-CEN10-131 resulted in a tumor growth inhibition of 99%, superior to the effects of Rituximab at 79%. Furthermore, only Rituximab-CEN10-131 provided a complete eradication of the tumor burden (2/6 mice showing no palpable tumors on day 45). Finally, EDC9 conjugate dose response was evaluated by dosing the models with 0.5, 1.5, or 5 mg/kg (Figure 6B). The results demonstrate clear dose dependence in inhibition of tumor growth. It showed be noted that Rituximab-CEN10-131 shows anti-tumor efficacy at doses at and below the preliminary maximum tolerated dose in cynomolgus monkey as discussed in Example 5.

**Example 7. In vivo efficacy of a single dose of Rituximab-CEN10-131.**

This study was performed to examine the efficacy of a single dose of Rituximab-CEN10-131 relative to a control EDC that does not bind to CD20, prepared according to Example 1. The data presented in Figures 7A-7C show clear antitumor activity of Rituximab-CEN10-131 and virtually no difference between the control EDC and the vehicle control groups even at the relatively high dose of 25 mg/kg - again demonstrating the clear need that the targeting moiety of EDC9 binds CD20.

**Example 8. In vitro cytotoxicity of EDC9 conjugates compared to their unconjugated components**

In vitro cytotoxicity of individual components of EDC9 were examined in isolation, i.e. the targeting moiety (Rituximab), linker-drug (CEN10-131), and the drug (CEN09-106) and compared Rituximab-CEN10-131 (an EDC9) and a Control EDC including a targeting moiety that does not bind to CD20. The data presented in Figure 8 demonstrates that covalent attachment of the three components of Rituximab-CEN10-131 is essential to the potency of the EDC9. More specifically, the data show that unconjugated CEN10-106, even the presence of Rituximab, is 2600X less potent than the corresponding EDC9 conjugate (Rituximab-CEN10-131). In addition, the Control EDC, including CEN10-131 conjugated to an antibody preparation that shows no binding to Ramos cells, is essentially inactive, demonstrating the requirement that the targeting moiety bind CD20 expressed on Ramos cells.

## Claims

1. An extracellular drug conjugate (EDC) comprising a targeting moiety portion that binds CD20, a drug portion that binds to an extracellular portion of an Na,K-ATPase, and a linker portion that covalently joins the targeting moiety portion to the drug portion, wherein the targeting moiety portion comprises an antibody that binds CD20, and wherein the drug portion comprises a bufadienolide, a cardiac glycoside or an aglycone of a cardiac glycoside.

2. The EDC of claim 1, wherein the drug portion comprises scillarenin or bufalin.

3. The EDC of any preceding claim, wherein the linker portion comprises a functional group, and a polyethylene glycol spacer of 12 to 36 units.

4. The EDC of any preceding claim, wherein the linker portion comprises a nitrogen heteroatom within 10 atomic bonds of the drug portion.

5. The EDC of any preceding claim, wherein the antibody comprises rituximab or a rituximab biosimilar.

6. The EDC of claim 1, wherein the targeting moiety comprises rituximab, the drug portion comprises scillarenin and an aminoglycoside covalently bound to the C3 oxygen substituent of scillarenin, and the linker portion comprises a polyethylene glycol spacer including 36 PEG units.

7. The EDC of claim 1, wherein the targeting moiety comprises rituximab, the drug portion comprises bufalin and a piperazine-carbamate moiety covalently bound to the C3 oxygen substituent of bufalin, and the linker portion comprises a polyethylene glycol spacer including 24 PEG units.

8. The EDC of claim 1, wherein the EDC is Rituximab-CEN10-131 or Rituximab-CEN-375.

9. The EDC of claim 8 having an antibody loading of about 1 to about 10, about 4 to about 8, or about 4 to about 6.

10. A composition for use in treating cancer or an inflammatory disease, the composition comprising an EDC of any one of claims 1 to 9.

11. The composition for use of claim 10, wherein the cancer expresses CD20.

12. The composition for use of claim 10 or claim 11, wherein the cancer is a lymphoma or leukemia.

13. A composition for use in a method of treating a subject resistant to Rituximab therapy, the composition comprising an EDC of any one of claims 1 to 9, wherein the method comprises identifying a subject as receiving Rituximab therapy and as no longer responding to such therapy, discontinuing the Rituximab therapy, and thereafter administering said composition.

## Patentansprüche

1. Extrazelluläres Arzneimittelkonjugat (EDC), das einen CD20 bindenden Targetierungsanteil, einen sich an einen extrazellulären Teil einer Na,K-ATPase bindenden Arzneimittelteil und einen den Targetierungsanteil kovalent mit

dem Arzneimittelteil verbindenden Linkerteil umfasst, wobei der Targetierungsanteil einen CD20 bindenden Antikörper umfasst und wobei der Arzneimittelteil ein Bufadienolid, ein Herzglycosid oder ein Aglykon eines Herzglycosids umfasst.

**2.** EDC nach Anspruch 1, wobei der Arzneimittelteil Scillarenin oder Bufalin umfasst.

**3.** EDC nach einem vorherigen Anspruch, wobei der Linkerteil eine funktionelle Gruppe und einen Polyethylenglykol-Spacer mit 12 bis 36 Einheiten umfasst.

**4.** EDC nach einem vorherigen Anspruch, wobei der Linkerteil ein Stickstoffheteroatom innerhalb von 10 Atombindungen des Arzneimittelteils umfasst.

**5.** EDC nach einem vorherigen Anspruch, wobei der Antikörper Rituximab oder ein Rituximab-Biosimilar umfasst.

**6.** EDC nach Anspruch 1, wobei der Targetingsanteil Rituximab umfasst, der Arzneimittelteil Scillarenin und ein kovalent an den C3-Sauerstoffsubstituenten von Scillarenin gebundenes Aminoglycosid umfasst und der Linkerteil einen Polyethylenglycol-Spacer mit 36 PEG-Einheiten umfasst.

**7.** EDC nach Anspruch 1, wobei der Targetingsanteil Rituximab umfasst, der Arzneimittelteil Bufalin und einen kovalent an den C3-Sauerstoffsubstituenten von Bufalin gebundenen Piperazin-Carbamat-Anteil umfasst und der Linkerteil einen Polyethylenglykol-Spacer mit 24 PEG-Einheiten umfasst.

**8.** EDC nach Anspruch 1, wobei das EDC Rimximab-CEN10-l3l oder Rituximab-CEN-375 ist.

**9.** EDC nach Anspruch 8 mit einer Antikörperbeladung von etwa 1 bis etwa 10, etwa 4 bis etwa 8 oder etwa 4 bis etwa 6.

**10.** Zusammensetzung zur Verwendung bei der Behandlung von Krebs oder einer Entzündungskrankheit, wobei die Zusammensetzung ein EDC nach einem der Ansprüche 1 bis 9 umfasst.

**11.** Zusammensetzung zur Verwendung nach Anspruch 10, wobei der Krebs CD20 exprimiert.

**12.** Zusammensetzung zur Verwendung nach Anspruch 10 oder Anspruch 11, wobei der Krebs ein Lymphom oder Leukämie ist.

**13.** Zusammensetzung zur Verwendung in einem Verfahren zur Behandlung eines Subjekts, das gegen Rituximab-Therapie resistent ist, wobei die Zusammensetzung ein EDC nach einem der Ansprüche 1 bis 9 umfasst, wobei das Verfahren das Identifizieren eines Subjekts als eine Rituximab-Therapie erhaltend und nicht mehr auf eine solche Therapie ansprechend, das Absetzen der Rituximab-Therapie und das anschließende Verabreichen der genannten Zusammensetzung beinhaltet.

**Revendications**

**1.** Conjugué de médicament extracellulaire (EDC) comprenant une partie de fraction de ciblage qui se lie à CD20, une partie de médicament qui se lie à une partie extracellulaire d'une Na,K-ATPase, et une partie de liaison qui joint de manière covalente la partie de fraction de ciblage à la partie de médicament, dans lequel la partie de fraction de ciblage comprend un anticorps qui se lie à CD20, et dans lequel la partie de médicament comprend un bufadiénolide, un glycoside cardiaque ou un aglycone de glycoside cardiaque.

**2.** L'EDC selon la revendication 1, dans lequel la partie de médicament comprend de la scillarénine ou de la bufaline.

**3.** L'EDC selon l'une quelconque des revendications précédentes, dans lequel la partie de liaison comprend un groupe fonctionnel, et un espaceur polyéthylène glycol de 12 à 36 unités.

**4.** L'EDC selon l'une quelconque des revendications précédentes, dans lequel la partie de liaison comprend un hétéroatome d'azote dans les 10 liaisons atomiques de la partie de médicament.

**5.** L'EDC selon l'une quelconque des revendications précédentes, dans lequel l'anticorps comprend du rituximab ou

un agent biosimilaire au rituximab.

6. L'EDC selon la revendication 1, dans lequel la fraction de ciblage comprend du rituximab, la partie de médicament comprend de la scillarénine et un aminoglycoside lié de manière covalente au substituant oxygène en C3 de la scillarénine, et la partie de liaison comprend un espaceur polyéthylène glycol comprenant 36 unités de PEG.

7. L'EDC selon la revendication 1, dans lequel la fraction de ciblage comprend du rituximab, la partie de médicament comprend de la bufaline et une fraction de pipérazinecarbamate liée de manière covalente au substituant oxygène en C3 de la bufaline, et la partie de liaison comprend un espaceur polyéthylène glycol comprenant 24 unités de PEG.

8. L'EDC selon la revendication 1, où l'EDC est du rituximab-CEN10-131 ou du rituximab-CEN-375.

9. L'EDC selon la revendication 8, ayant une charge d'anticorps d'environ 1 à environ 10, d'environ 4 à environ 8, ou d'environ 4 à environ 6.

10. Composition pour une utilisation dans le traitement d'un cancer ou d'une maladie inflammatoire, la composition comprenant un EDC selon l'une quelconque des revendications 1 à 9.

11. Composition pour une utilisation selon la revendication 10, où le cancer exprime CD20.

12. Composition pour une utilisation selon la revendication 10 ou la revendication 11, où le cancer est un lymphome ou une leucémie.

13. Composition pour une utilisation dans une méthode de traitement d'un sujet résistant à une thérapie au rituximab, la composition comprenant un EDC selon l'une quelconque des revendications 1 à 9, où la méthode comprend identifier un sujet comme recevant une thérapie au rituximab et ne répondant plus à une telle thérapie, arrêter la thérapie au rituximab et par la suite administrer ladite composition.

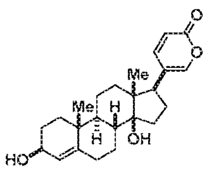

Scillarenin

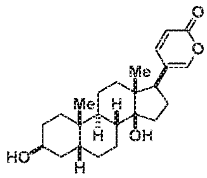

Bufalin

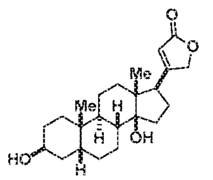

Digitoxigenin

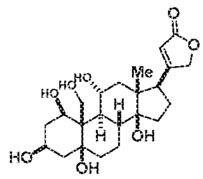

Ouabagenin

# Figure 1A

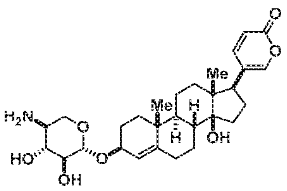

CEN09-106

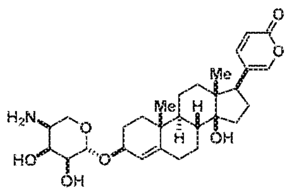

CEN09-107

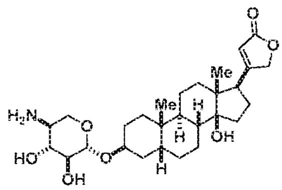

CEN-319

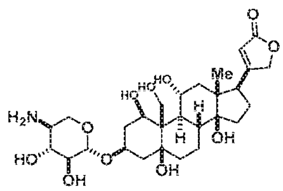

CEN-317

# Figure 1B

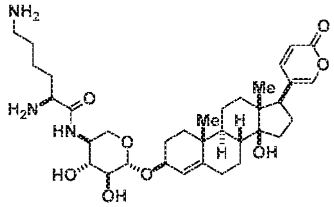

CEN10-110

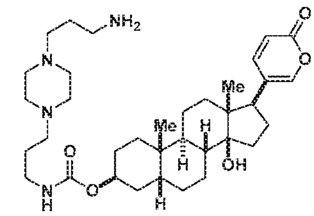

CEN-371

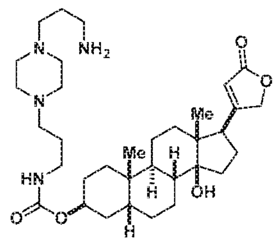

CEN-381

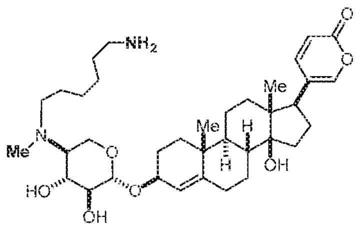

Scillarenin-4'-*N*-methylhexane-1,6-diamino-4'-deoxy-β-L-xylopyranoside

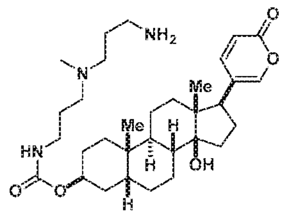

CEN-372

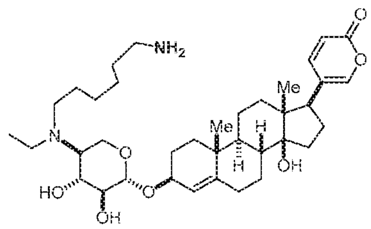

Scillarenin-4'-*N*-ethylhexane-1,6-diamino-4'-deoxy-β-L-xylopyranoside

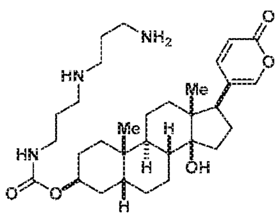

CEN-373

# Figure 1C

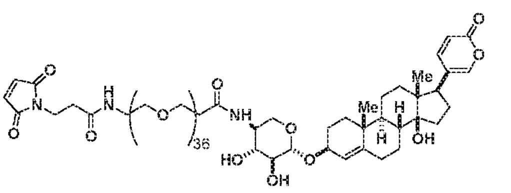

CEN10-131
(maleimide-PEG36-CEN09-106)

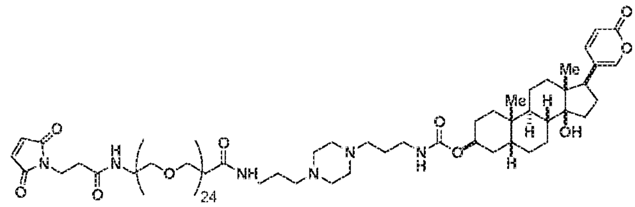

CEN-375
(maleimide-PEG24-CEN-371)

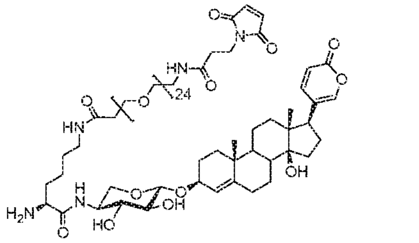

CEN-301
(maleimide-PEG24-CEN10-110)

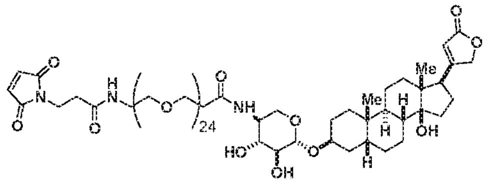

CEN-320
(maleimide-PEG24-CEN-319)

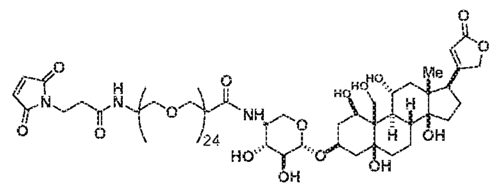

CEN-318
(maleimide-PEG24-CEN-317)

# Figure 1D

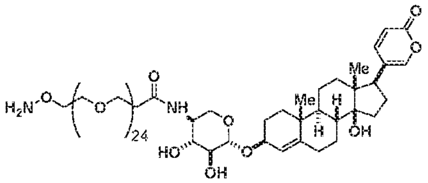

CEN-332
(aminooxy-PEG24-CEN09-106)

# Figure 1E

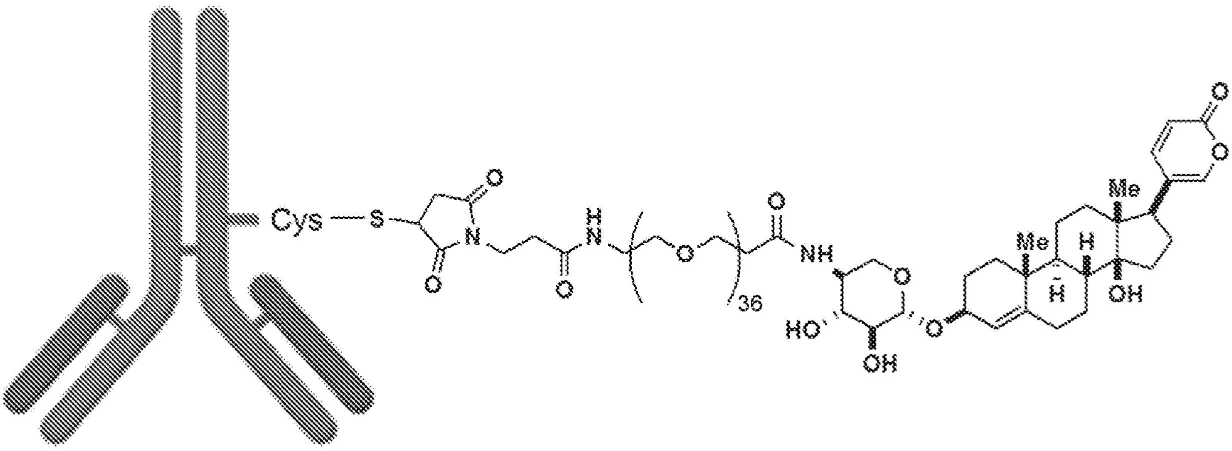

Figure 1F

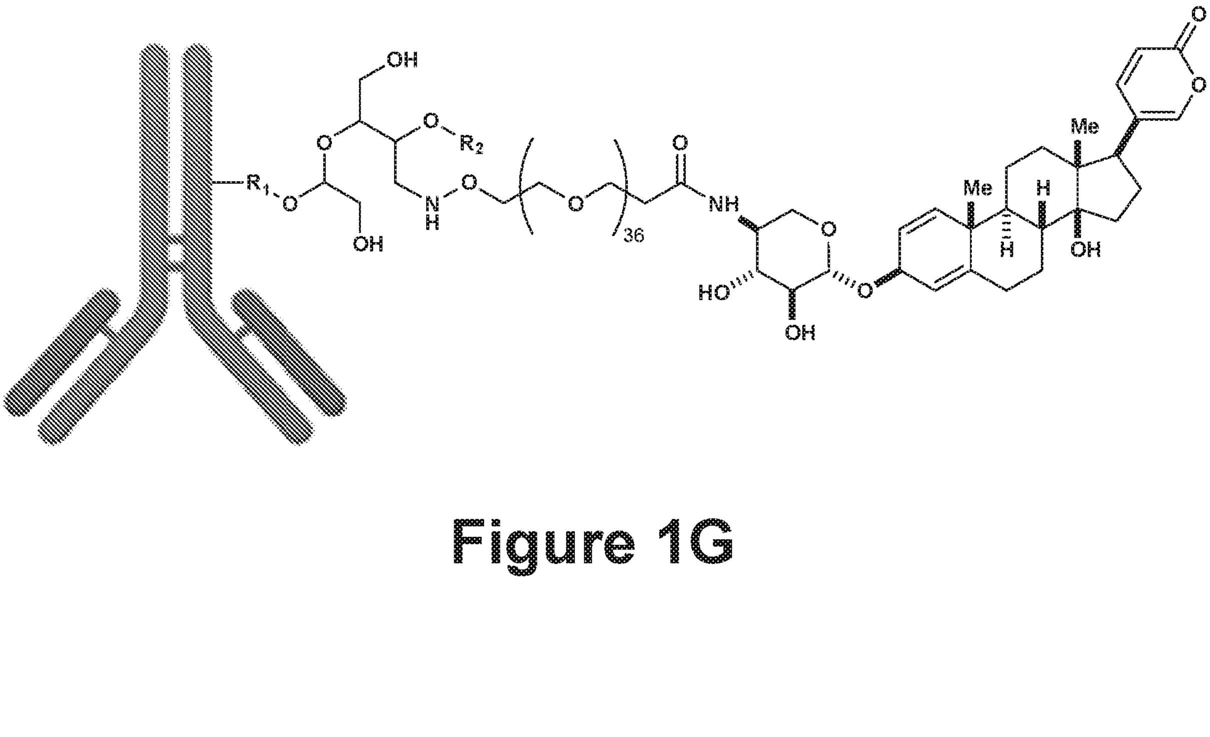

Figure 1G

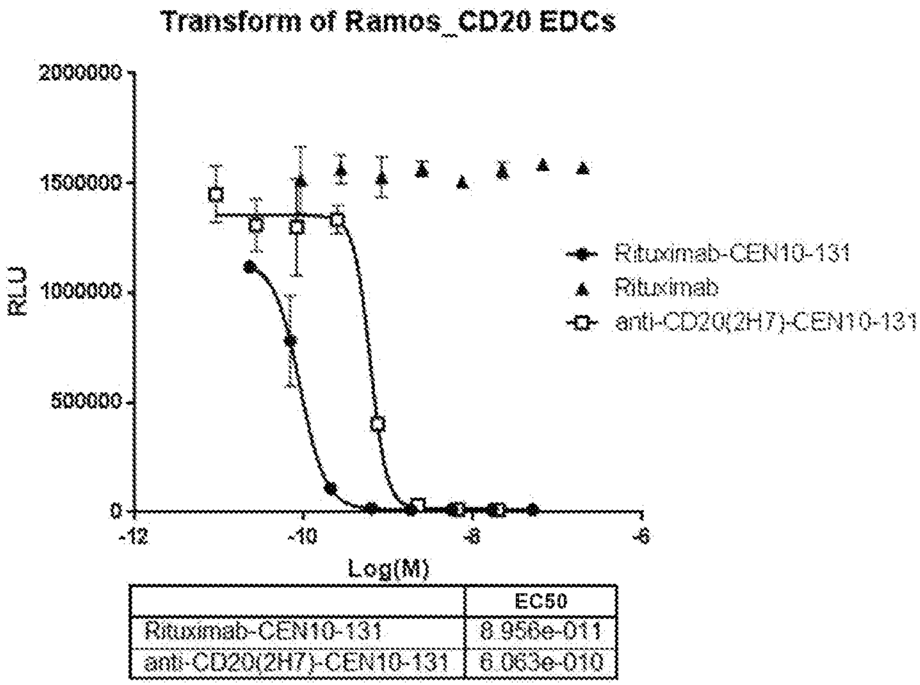


| | EC50 |
|---|---|
| Rituximab-CEN10-131 | 8.956e-011 |
| anti-CD20(2H7)-CEN10-131 | 6.063e-010 |

# Figure 2

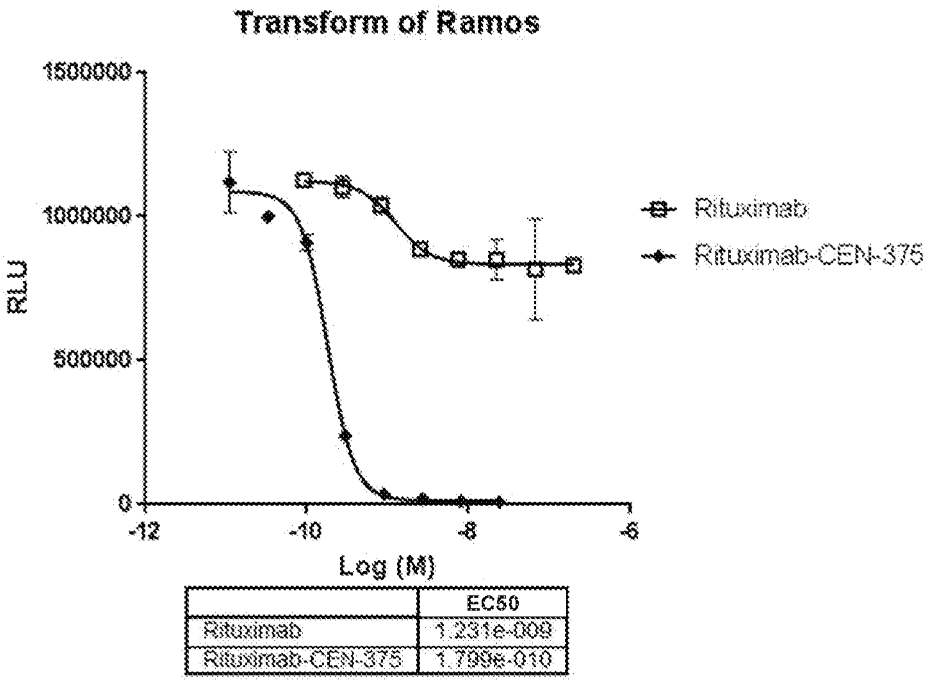


| | EC50 |
|---|---|
| Rituximab | 1.231e-009 |
| Rituximab-CEN-375 | 1.799e-010 |

# Figure 3

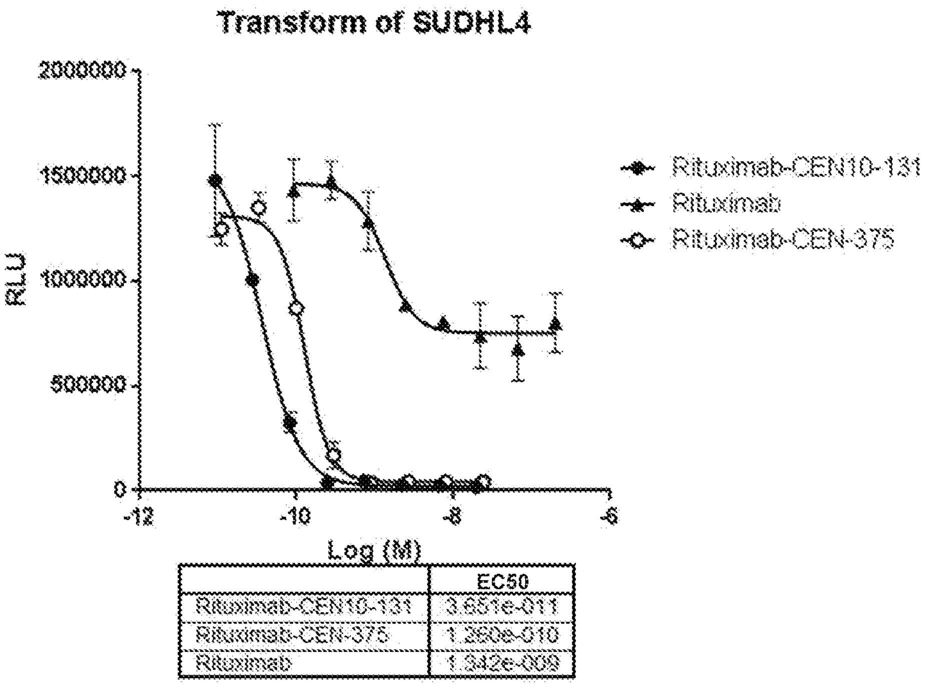


| | EC50 |
|---|---|
| Rituximab-CEN10-131 | 3.651e-011 |
| Rituximab-CEN-375 | 1.260e-010 |
| Rituximab | 1.342e-009 |

# Figure 4

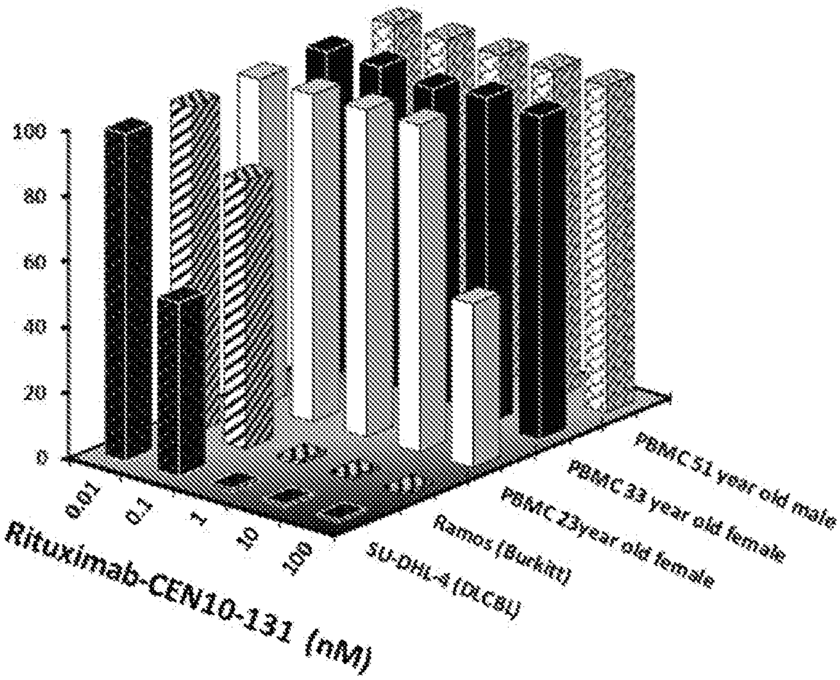


# Figure 5

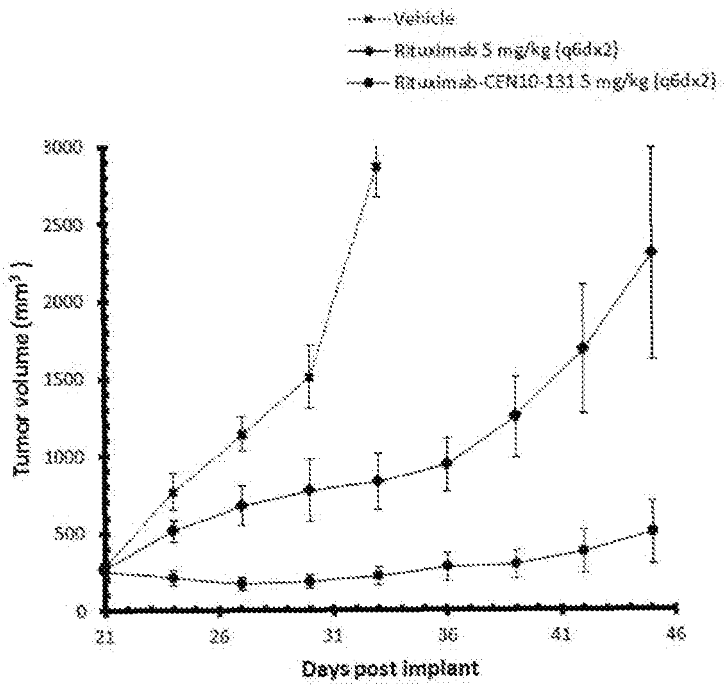


Figure 6A

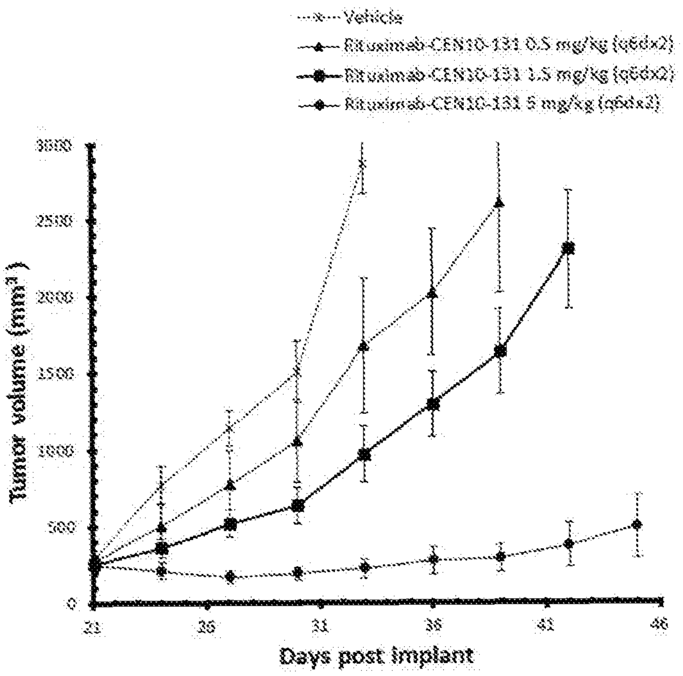


Figure 6B

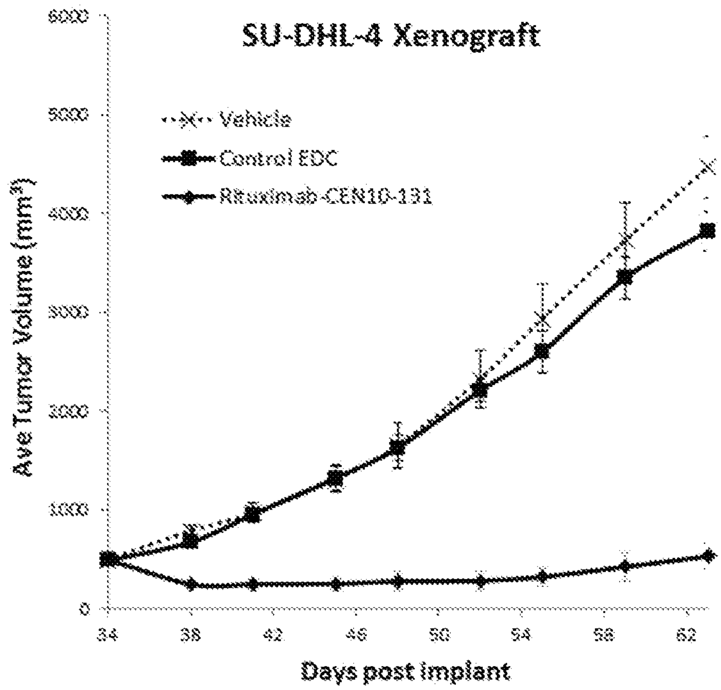


Figure 7A

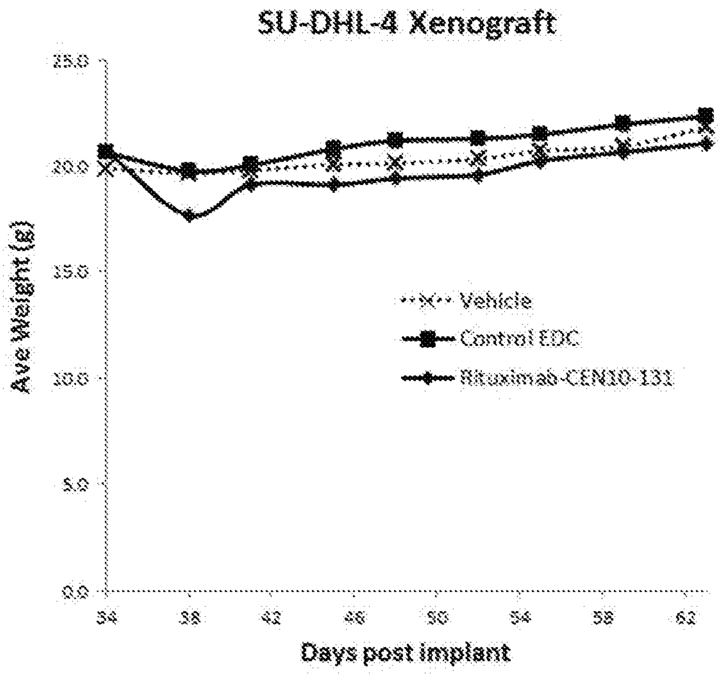


Figure 7B

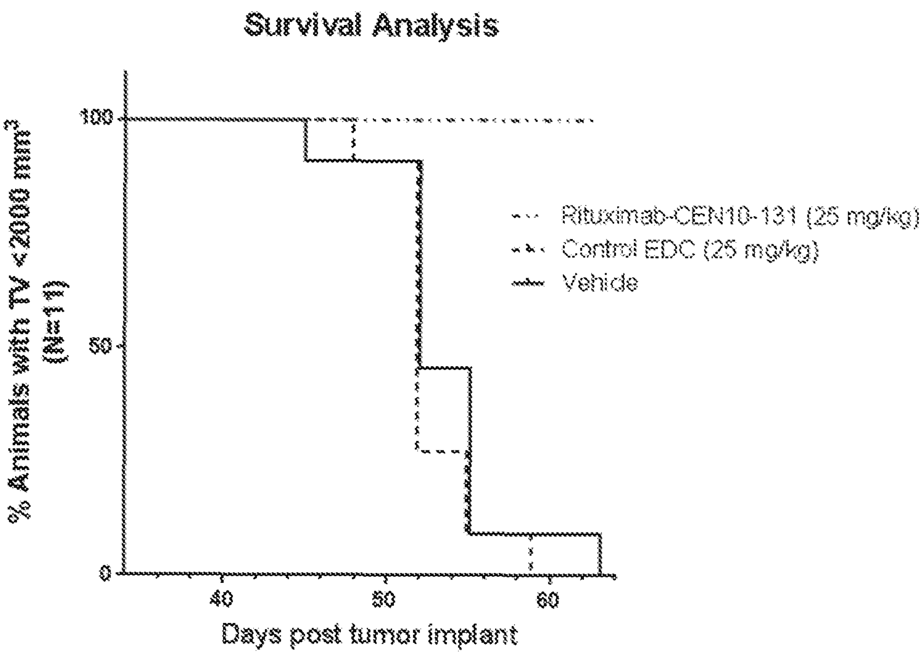


# Figure 7C

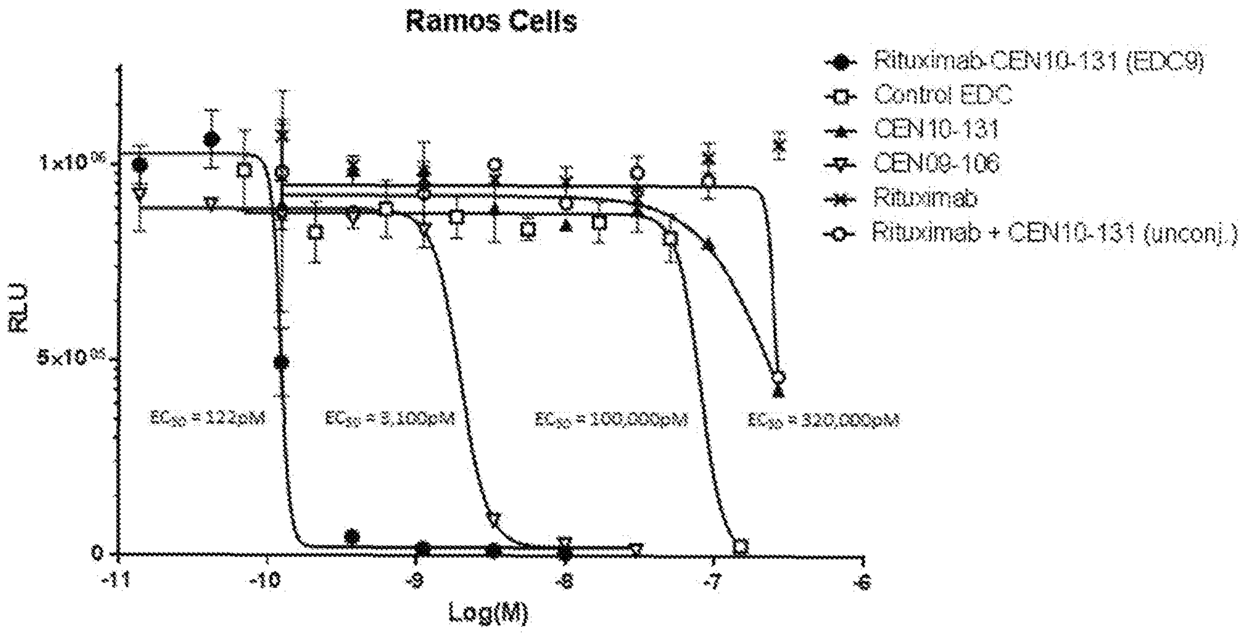


# Figure 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2011031870 W
- US 8470980 B
- US 9364554 B
- US 2010017480 W
- US 8703723 B
- US 2012122514 W
- US 2014193436 A
- US 2012178173 W
- US 2015123687 W
- WO 9404678 A
- US 5585089 A, Queen
- US 5693761 A
- US 5693762 A
- US 5225539 A, Winter


### Non-patent literature cited in the description


- *Cancer Res,* 15 March 2009, vol. 2009 (6), 69
- *Haematologica,* 2009, vol. 94, 423-7
- *Nature Medicine,* 2003, vol. 9 (1), 129-134
- Cancer Principles and Practice of Oncology. Lippincott Williams & Wilkins, 2005
- **WARD et al.** *Nature,* 1989, vol. 341, 544-546
- *Cancer Res,* 15 March 2009, vol. 69 (6
- **DURCY, L.** *Bioconjugate Chem.,* 2010, vol. 21, 5-13
- **LAURSEN et al.** *PNAS,* 2015, vol. 112 (6), 1755-60